# FASCICULE DE BREVET EUROPEEN

(11) **EP 2 844 753 B1**
(45) Date de publication et mention de la délivrance du brevet: **13.02.2019**
(21) Numéro de dépôt: 13723042.1
(22) Date de dépôt: 02.05.2013
(51) Int. Cl.: C12Q 1/37, C07K 1/36, C07K 1/107, G01N 33/68, C12N 13/00, C07K 1/12

(54) **PROCÉDÉ D'OBTENTION DE PEPTIDES**
VERFAHREN ZUR GEWINNUNG VON PEPTIDEN
METHOD FOR OBTAINING PEPTIDES

(30) Priorité: 03.05.2012 FR 1254090
(43) Date de publication de la demande: 11.03.2015
(73) Titulaire: bioMérieux, 69280 Marcy-l'Etoile (FR); Commissariat à l'Energie Atomique et aux Energies Alternatives, 75015 Paris (FR)
(72) Inventeur: CHARLES, Marie-Hélène, F-69420 Condrieu (FR); CHARRIER, Jean-Philippe, F-69160 Tassin La Demi-Lune (FR); CUBIZOLLES, Myriam-Laure, F-38700 Corenc (FR); DUPONT-FILLIARD, Agnès, F-38190 Les Adrets (FR); LANET, Véronique, F-69007 Lyon (FR); RIVERA, Florence, F-38800 Champagnier (FR); VERON, Laurent, F-69100 Villeurbanne (FR); BAUJARD-LAMOTTE, Lucie, 75017 Paris (FR); PEPONNET, Christine, 38170 Seyssinet (FR)
(74) Mandataire: Murgitroyd & Company
(86) Numéro de dépôt international: PCT/EP2013/059192
(87) Numéro de publication internationale: WO 2013/164427

(56) Documents cités:
- WO-A1-2009/072728
- WO-A1-2009/082044
- SANTOS H M ET AL: "An improved clean sonoreactor-based method for protein identification by mass spectrometry-based techniques", TALANTA, ELSEVIER, AMSTERDAM, NL, vol. 77, no. 2, 15 décembre 2008 (2008-12-15), pages 870-875, XP025586417, ISSN: 0039-9140, DOI: 10.1016/J.TALANTA.2008.07.048 [extrait le 2008-08-03]
- SANTOS H M ET AL: "Ultrasonic multiprobe as a new tool to overcome the bottleneck of throughput in workflows for protein identification relaying on ultrasonic energy", TALANTA, ELSEVIER, AMSTERDAM, NL, vol. 81, no. 1-2, 15 avril 2010 (2010-04-15), pages 55-62, XP026923771, ISSN: 0039-9140, DOI: 10.1016/J.TALANTA.2009.11.033 [extrait le 2009-11-18]

## Description

### Domaine technique

La présente invention concerne un procédé d'obtention de peptides à partir de cellules procaryotes et/ou eucaryotes en vue de leur analyse ultérieure.

L'analyse de ces peptides peut en effet s'avérer utile dans un grand nombre d'applications, notamment dans la recherche de biomarqueurs ou l'analyse peptidique bactérienne, par exemple dans le cadre d'applications d'identification, typage, détection de marqueurs de résistance et de virulence, et ce dans le domaine médical, pharmaceutique et agroalimentaire.

### Etat de la technique

Les protocoles d'extraction et de purification de peptides selon l'art antérieur sont généralement très longs. De surcroît ces méthodes sont, en général, manuelles et complexes à mettre en oeuvre.

La demande internationale WO 2006/031063 décrit des compositions visant à hydrolyser des polypeptides, cette réaction d'hydrolyse étant obtenue de manière chimique par l'utilisation d'une solution dite « PCA », comprenant un acide (tel que l'acide acétique pur), un solvant organique miscible à l'eau (tel que l'acétonitrile) et un agent réducteur (tel que le tris(2-carboxyéthyl)phosphine). Cette digestion chimique est réalisée à 99,9°C pendant deux heures. Toutefois WO 2006/031063 prévoit, de façon facultative, de coupler cette étape de digestion chimique à une étape de digestion enzymatique préalable, possiblement afin d'augmenter l'efficacité de la protéolyse. L'étape de digestion enzymatique est effectuée à 37°C pendant 12 heures en utilisant une trypsine modifiée, postérieurement à une étape de dénaturation thermique (90°C pendant 20 minutes). L'étape de digestion chimique susmentionnée est réalisée suite à cette étape de digestion enzymatique (99,9°C pendant 2 heures). Le temps total de traitement de l'échantillon protéique est donc supérieur à 14 heures, ce qui représente un inconvénient majeur indiscutable. En outre, WO 2006/031063 enseigne l'utilisation d'une trypsine modifiée, plus coûteuse qu'une trypsine conventionnelle.

La demande internationale WO 2008/128029 divulgue, quant à elle, un procédé de fragmentation protéique ou peptidique en solution, ledit procédé comprenant, postérieurement à l'étape de digestion enzymatique ou chimique initiale, une série d'étapes additionnelles de séparation et de fractionnement. Ces dernières contribuent d'une part à augmenter le temps du procédé de fractionnement dans son ensemble et, d'autre part, requièrent diverses manipulations, rendant ainsi extrêmement difficile l'automatisation de ce procédé.

Le brevet US 7,622,273 décrit un protocole visant à altérer les polypeptides ou protéines modifiés post-traduction, ces peptides et protéines étant directement liés à une puce à protéines et ledit protocole comprenant les étapes de traitement chimique, digestion enzymatique ou chimique et d'identification subséquente de protéines sur lesdites puces à protéines. L'étape de traitement chimique comprend la dénaturation, la réduction et l'alkylation desdites protéines tandis que celle de digestion enzymatique inclut la déglycosylation et/ou la déphosphorylation desdites protéines et la digestion de ces dernières par protéolyse chimique ou enzymatique. Toutes les réactions sont effectuées de façon séquentielle sur puce à protéines. L'étape de traitement chimique dure a minima 3 heures et 15 minutes et celle de digestion enzymatique deux heures (cf. Exemple 1), soit une durée minimale de 5 heures et 15 minutes. Celle-ci, bien qu'inférieure à la durée des méthodes traditionnelles - approximativement 24 heures - demeure néanmoins insatisfaisante, notamment eu égard aux exigences inhérentes aux domaines clinique ou pharmaceutique. Qui plus est, les échantillons complexes (plasma, urine, liquide céphalorachidien, etc.) peuvent exiger des stratégies de fractionnement ou d'épuisement pour isoler les protéines cibles préalablement à la mise en oeuvre du protocole divulgué dans ce brevet, ce qui augmente d'autant la durée totale de traitement desdits échantillons.

La demande internationale WO 2011/130521 décrit une méthode de digestion protéolytique, laquelle utilise une gamme de cycles de pression (par exemple de 5 à 35 kpsi, à savoir environ de 344,74 bars à 2413,16 bars) afin de réduire la durée de cette méthode. Même si cette durée semble diminuer par rapport aux méthodes de l'art antérieur, il n'en demeure pas moins que le temps cumulé correspondant aux étapes de réduction (10mM de DTT à 37°C pendant 1 heure) et d'alkylation (50 mM d'iodoacétamide à température ambiante, dans l'obscurité, pendant 45 minutes) ne peut être inférieur à 1 heure et 45 minutes. Si l'on ajoute à ceci le temps de lyse des micro-organismes (3 minutes à 4500 rpm), le temps de manipulation du lysat avant l'étape de réduction (non précisé) et celui inhérent à la dilution de la solution avant l'étape de digestion enzymatique sous pression (également non-précisé), il est manifeste que la durée totale du protocole objet de WO 2011/130521 ne peut être inférieure à 2 heures, ce qui demeure insatisfaisant. En raison des fortes pressions utilisées, cette méthode requiert un appareillage complexe, apte à résister à ces fortes pressions, délicat à mettre en oeuvre et onéreux. Qui plus est, ladite méthode nécessite des étapes supplémentaires de filtration afin de réduire les concentrations très importantes en agent chaotropique (urée à 8M), préalablement à l'étape de digestion enzymatique afin de ne pas risquer l'inactivation de l'enzyme utilisée En effet, lorsqu'utilisés à forte concentration (à partir d'1 M), les agents chaotropiques employés lors de l'étape de dénaturation des protéines - tels que l'urée - vont également dénaturer la structure protéique de la ou des enzyme(s) utilisée(s) lors de l'étape de protéolyse enzymatique et vont avoir pour conséquence d'inactiver totalement ou partiellement cette enzyme. Afin d'éviter ceci, il est nécessaire, avant l'étape de protéolyse enzymatique (digestion enzymatique), d'effectuer des étapes additionnelles de dilution et/ou filtration, ce qui nécessite un laps de temps additionnel et complexifie l'automatisation du procédé dans son ensemble. Une alternative consiste à utiliser des enzymes génétiquement modifiées (par exemple des trypsines modifiées) capables d'effectuer une digestion protéique en présence de concentrations importantes en agent(s) chaotropique(s). Toutefois, ces enzymes modifiées possèdent des cinétiques d'action moins rapides que celles des enzymes natives et ont un coût d'achat bien supérieur à celui de ces dernières.

Tout comme WO 2011/130521, Santos H. M. et al. **[26]** requiert un appareillage complexe, apte à résister à de fortes pressions, délicat à mettre en oeuvre et onéreux. En effet, cette publication divulgue un procédé comprenant la digestion de protéines split-soret cytochrome c de *D. desulfuricans* ATCC27774 et l'identification de ces protéines par empreinte protéique (« peptide mass fingerprint », en langue anglaise), ledit procédé comprenant une étape de lyse sous « French press » sous une pression de 9000 psi (soit environ 620,26 bars).

Il existe donc un besoin de mettre au point un protocole permettant l'obtention de peptides à partir de cellules procaryotes et/ou eucaryotes qui résout tout ou partie des problèmes susvisés, à savoir un protocole efficace, rapide, facilement automatisable et ne nécessitant pas d'étapes superflues de dilution.

### Exposé de l'invention

Ainsi un objet de la présente invention concerne un procédé d'obtention de peptides à partir de cellules procaryotes et/ou eucaryotes, ledit procédé comprenant les étapes suivantes :
a) lyse des cellules procaryotes et/ou eucaryotes et récupération des protéines ainsi obtenues,
b) dénaturation desdites protéines en utilisant au moins un agent dénaturant,
c) alkylation des protéines dénaturées en utilisant au moins un agent alkylant,
d) protéolyse enzymatique des protéines obtenues à l'issue de l'étape c) en utilisant au moins une enzyme protéolytique,
e) récupération des peptides obtenus à l'issue de l'étape de protéolyse enzymatique d),
dans lequel la lyse des cellules procaryotes et/ou eucaryotes à l'étape a) est une lyse à faible concentration en agent(s) chaotropique(s),
et dans lequel au moins les étapes a) et b) sont effectuées conjointement ;
ladite lyse à faible concentration en agent(s) chaotropique(s) étant réalisée à une concentration en agent(s) chaotropique(s) inférieure ou égale à 1M, de préférence inférieure ou égale à 100 mM, et
ledit procédé étant mis en oeuvre sous une pression inférieure à 100 bars, de préférence inférieure à 50 bars, avantageusement inférieure à 10 bars.

Ce procédé (protocole) d'obtention de peptides est applicable aux cellules procaryotes (par exemple aux bactéries), aux cellules eucaryotes (cellules humaines, animales, levures, etc) ou à un mélange de cellules procaryotes et eucaryotes.

Selon un mode de réalisation préféré, l'on utilisera le procédé selon la présente invention afin d'obtenir des peptides à partir de cellules procaryotes, de préférence à partir de bactéries (Gram+ ou Gram-).

Par « agent dénaturant », l'on entend un agent - physique ou chimique - capable d'induire un phénomène de dénaturation des protéines et polypeptides ; ces derniers quittant leur état natif et perdant peu à peu leur structure secondaire, tertiaire et quaternaire. La dénaturation peut parfois être réversible, le retour à l'état natif étant alors possible et l'activité de la protéine étant restaurée.

La dénaturation des protéines est due à la sensibilité de ces dernières en fonction de leur environnement physico-chimique. Les protéines se dénaturent lorsque les interactions entre les résidus sont perturbées par un agent dénaturant. Les liaisons covalentes entre acides aminés adjacents de la chaine polypeptidiques ne sont pas rompues. En revanche, certaines conditions de dénaturation peuvent entrainer la rupture de liaisons disulfure entre résidus cystéine non adjacents de la chaine polypeptidiques et qui assurent la stabilité globale de la structure quaternaire de la protéine.

A titre d'agent dénaturant, l'on distingue principalement :
a) les agents physiques, tels que la température ; l'augmentation de la température engendre en effet une agitation thermique des atomes de la molécule ; celle-ci provoque une rupture des interactions faibles, comme les liaisons hydrogène, qui stabilisent la structure spatiale ;
b) les agents chimiques, tels que :
   - les acides et bases ; en modifiant le pH environnant ils induisent une modification des charges portées par les groupements ionisables et altèrent donc les liaisons ioniques et hydrogènes stabilisant la structure spatiale de la protéine ;
   - les agents chaotropiques comme l'urée, les sels de guanidine (par exemple l'hydrochlorure de guanidine ou le perchlorate de lithium) ; utilisés à fortes concentrations, ces composés fragilisent fortement les liaisons hydrogène des protéines (principales liaisons de faible énergie responsables du maintien des structures secondaire, tertiaire et quaternaire des protéines) ;
   - les agents réducteurs de thiols comme le 2-mercaptoéthanol ou le dithiothreitol (DTT) ; ils permettent le clivage des ponts disulfures et contribuent ainsi à fragiliser la structure tertiaire ou quaternaire des protéines ;
   - les détergents, qui agissent par modification de l'interaction avec le solvant aqueux (par exemple le sodium dodecyl sulfate - plus connu sous l'acronyme « SDS »).

Certains agents dénaturants peuvent s'avérer non réversibles, tels que les métaux lourds (Pb, Hg, etc.) et certains acides (par exemple HNO₃, acide trichloracétique, etc.

Parmi les agents chaotropiques, l'on opère une distinction, aux fins de la présente invention, entre les agents chaotropiques dits « salins », tels que les sels de guanidine (ou de guanidinium) et les agents chaotropiques dits « non salins », tels que l'urée.

Par « lyse à faible concentration en « agent(s) chaotropique(s) », l'on entend une lyse réalisée à une concentration en agent(s) chaotropique(s) inférieure ou égale à 1M, de préférence inférieure ou égale à 100 mM.

En effet, à de telles concentrations, les agents dits « chaotropiques » n'exercent plus d'activité dénaturante sur les protéines et polypeptides, perdant ainsi leur propriété chaotropique.

Cette solution technique s'oppose aux solutions traditionnelles présentées dans l'art antérieur, lesquelles emploient généralement des agents chaotropiques et plus particulièrement des sels de guanidine (tels que de l'hydrochlorure de guanidine, guanidine thiocyanate, etc.) - à des concentrations généralement très élevées, de l'ordre de 6M. De telles concentrations en agents chaotropiques - et notamment en sels (par exemple en sels de guanidine) - perturbent fortement l'étape de protéolyse enzymatique , en particulier lorsque cette dernière est effectuée en utilisant une protéase à sérine telle que la trypsine. Le fait d'effectuer, dans le cadre du procédé d'obtention de peptides selon la présente invention, une lyse à faible concentration en agent(s) chaotropique(s) permet non seulement d'améliorer l'efficacité de l'étape de protéolyse d) mais également d'éviter de devoir recourir à des étapes de filtration/dilution additionnelles avant la susdite étape de protéolyse. Le gain de temps ainsi réalisé permet d'obtenir un protocole significativement plus rapide que ceux décrits dans l'état de la technique mais également plus facilement automatisable.

En outre, cette faible concentration en agent(s) chaotropique(s) autorise l'emploi d'enzymes protéolytiques natives et ne nécessite pas de recourir à des enzymes protéolytiques génétiquement modifiées, lesquelles possèdent des cinétiques d'action moins rapides que celles des enzymes natives et présentent un coût d'achat bien supérieur au coût d'achat de ces dernières.

Selon un mode de réalisation préféré, l'on effectue une lyse à faible concentration en sel(s), ce qui signifie que la lyse est réalisée à une concentration saline inférieure ou égale à 50 mM, de préférence inférieure ou égale à 30 mM.

Avantageusement, la lyse à faible concentration en agent(s) chaotropique(s) est réalisée en l'absence d'agent(s) chaotropique(s) non salin(s) tel(s) que l'urée. En effet, le procédé selon la présente invention ne requiert pas l'emploi de tels agents chaotropiques non salins.

A titre de lyse à faible concentration en agent(s) chaotropique(s), l'on utilise préférablement le protocole universel de lyse cellulaire de procaryotes et/ou d'eucaryotes décrit au sein de la demande internationale WO 02/10333. Ce protocole universel consiste en un procédé de lyse cellulaire de procaryotes et/ou d'eucaryotes ou de lyse cellulaire simultanée de procaryotes et d'eucaryotes qui consiste à adapter au moins trois des paramètres suivants :
- un pourcentage en masse de billes actives (billes lysantes) de petit diamètre par rapport à des billes actives (billes lysantes) de grand diamètre inférieur ou égal à 50 %, et/ou
- une masse totale de billes lysantes (actives), comprenant un mélange ou pas de billes de petit diamètre et/ou de billes de grand diamètre, compris entre 50 et 100 % par rapport à la masse totale de l'échantillon biologique traité, et/ou
- une durée de lyse comprise entre 10 et 20 mn, et/ou
- un nombre de billes de verre non lysantes mais d'entraînement des billes lysantes (actives) inférieur à sept (7), et/ou -un nombre de billes de fer non lysantes mais d'entraînement des billes lysantes (actives) compris entre cinq (5) et quinze (15), en fonction de la technique utilisée :
- la sonication,
- le vortex mécanique, ou
- le vortex magnétique.

De préférence, les billes lysantes (actives) de petit diamètre sont d'un diamètre compris entre 90 et 150 µm et préférentiellement d'environ 100 µm, et les billes lysantes (actives) de grand diamètre sont d'un diamètre compris entre 400 et 600 µm et préférentiellement d'environ 500 µm.

Toujours de manière préférentielle, les billes lysantes (actives) sont en verre.

Selon un mode de réalisation préféré, toujours tel qu'indiqué dans la demande WO 02/10333, si l'on utilise la technique de vortex mécanique, le procédé consiste à effectuer la lyse selon les paramètres suivants :
- une durée de la lyse de 11 à 20 mn, préférentiellement de 15 à 20 mn et encore plus préférentiellement de 20 mn,
- un pourcentage de billes de 100 µm de diamètre inférieur à 50 %, préférentiellement inférieure à 30 %, et encore plus préférentiellement de 20 %,
- une masse totale de billes lysantes (actives) supérieure à 60 %, préférentiellement supérieure à 80 %, et encore plus préférentiellement de 100 % de la masse totale de l'échantillon biologique traité, et
- un nombre de billes de verre inférieur à sept (7) préférentiellement égal à un (1).

Si l'on utilise la technique de vortex magnétique, le procédé consiste, de préférence, à effectuer une lyse selon les paramètres suivants :
- une durée de la lyse de 12 à 20 mn, préférentiellement de 15 à 20 mn et encore plus préférentiellement de 20 mn,
- une masse totale de billes lysantes (actives) de 100 µm supérieure à 80 % et préférentiellement de 100 % de la masse totale de l'échantillon biologique traité, et
- un nombre de billes de fer compris entre cinq (5) à quinze (15) billes, préférentiellement de dix (10) billes de fer.

D'une manière générale, l'on utilise, aux fins de la présente invention, toutes techniques de lyse à faible concentration en agent(s) chaotropique(s) ne nécessitant pas de recourir à des pressions élevées de l'ordre de plusieurs centaines voire milliers de bars. En effet, un des objectifs de la présente invention est la mise au point d'un protocole d'obtention de peptides efficace, rapide et pouvant être mis en oeuvre à l'aide d'un appareillage standard, sans nécessiter l'utilisation d'un appareillage spécifiquement adapté pour résister à de fortes voire très fortes pressions, appareillage complexe à mettre en oeuvre et extrêmement coûteux.

Le procédé d'obtention de peptides selon la présente invention est mis en oeuvre sous une pression inférieure à 100 bars, de préférence inférieure à 50 bars, avantageusement inférieure à 10 bars. Selon un mode de réalisation préféré, le procédé selon la présente invention est réalisé sous pression atmosphérique.

Selon un mode de réalisation préféré de la présente invention, la lyse à faible concentration en agent(s) chaotropique(s) est une lyse par sonication effectuée au moyen d'une sonde à ultrasons. De préférence, cette lyse par sonication est effectuée au moyen de ladite sonde à ultrasons en présence d'un mélange de billes de verre de 1000 µm et de billes de zirconium de 100µm.

Dans le cadre d'une lyse par sonication, l'on applique, de préférence, le protocole de lyse par sonication tel que décrit dans la demande internationale WO 02/10333 (p. 3,1. 18-26), à savoir en utilisant les paramètres suivants :
- une durée de la lyse de 9 à 20 mn, préférentiellement de 12 à 18 mn et encore plus préférentiellement de 15 mn,
- un pourcentage de billes de 100 µm de diamètre compris entre 10 à 50% préférentiellement entre 20 et 30 % et encore plus préférentiellement de 20 %, et
- une masse totale de billes lysantes (actives) comprise entre 50 à 100 % de la masse totale de l'échantillon biologique traité, préférentiellement compris entre 75 et 90 % et préférentiellement entre 80 et 85 %.

Concernant l'appareillage et la méthodologie du protocole de lyse par sonication *per se,* l'on utilise, de manière avantageuse, une sonotrode externe telle que la sonotrode VialTweeter commercialisée par la société Hielscher. Une telle sonde consiste en un bloc vibrant percé de trous dans lesquels peuvent être insérés des tubes tels que des tubes Eppendorf de volume 1,5mL dans lesquels sont introduits les cellules à lyser en suspension dans un tampon 3mM Borate à pH8 ainsi que les billes de verre 1mm et zirconium 100µm (50mg de chaque). Le tube est fermé puis la sonotrode activée pendant 5 à 15 minutes, préférentiellement 10 minutes, à une amplitude comprise entre 50% et 100% de l'amplitude nominale (entre 5 et 10 watts pour chaque tube selon sa position sur le bloc), préférentiellement 100% et un rapport cyclique de 40% à 60%, préférentiellement de 50%.

La sélection d'un protocole de lyse par sonication ne relève en aucun cas d'un choix arbitraire. Bien au contraire, la demanderesse a découvert, de manière surprenante, que le rendement du procédé d'obtention de peptides selon la présente invention était meilleur lorsque l'étape de lyse a) était une étape de lyse par sonication.

En outre, la demanderesse a découvert que l'omission de l'étape de préchauffage de la sonde à ultrasons (activation de la sonde durant 1h, ce qui fait monter la température de l'ensemble du système vibrant à 95°C) ne nuisait pas à l'efficacité dudit procédé. Ceci s'avère être une caractéristique tout à fait intéressante dans la mesure où cette étape de préchauffage de la sonde à ultrasons diminue la durée de vie de cette dernière et nécessite un apport d'énergie supplémentaire.

En conséquence de quoi, de manière préférée, la sonde à ultrasons ne subit pas d'étape de préchauffage en préalable à la lyse par sonication.

En ce qui concerne l'étape de protéolyse enzymatique d), cette dernière est réalisée par action d'une enzyme protéolytique, de préférence une protéase à sérine, avantageusement sélectionnée dans le groupe consistant en la trypsine, la chymotrypsine et l'élastase. De préférence, ladite enzyme protéolytique est la trypsine.

Cette protéolyse enzymatique est particulièrement préférée par rapport aux traitements physico-chimiques (traitements avec du peroxyde d'hydrogène, du bromure de cyanogène, de l'acide trifluoroacétique, etc.) car elle préserve davantage la structure des protéines, s'avère plus facile à contrôler et clive les chaines peptidiques sur des sites spécifiques (en C-terminal des résidus lysine et arginine dans le cas de la trypsine). Par « protéolyse enzymatique », l'on entend l'action simple ou combinée d'une ou plusieurs enzymes dans des conditions de réaction appropriées. Les enzymes effectuant la protéolyse, appelées protéases, clivent les protéines de façon site-spécifique. En effet, chaque protéase reconnaît généralement une séquence spécifique d'acides aminés au sein de laquelle cette protéase effectue toujours le même clivage. Certaines protéases reconnaissent un seul acide aminé ou une séquence de deux acides aminés entre lesquels elles opèrent un clivage, d'autres protéases reconnaissent des séquences d'acides aminés plus longues. Ces protéases peuvent être des endoprotéases ou des exoprotéases. Parmi les protéases connues l'on peut citer, comme décrit dans le document WO 2005/098071 :
- les enzymes spécifiques comme la trypsine qui scinde la liaison peptidique au niveau du groupe carboxylique des résidus Arg et Lys, l'endolysine qui clive la liaison peptidique du groupe -CO des lysines, la chymotrypsine qui hydrolyse la liaison peptidique au niveau du groupe carboxylique des résidus aromatiques (Phe, Tyr et Trp), la pepsine qui coupe au niveau du groupe NH2 des résidus aromatiques (Phe, Tyr et Trp), la protéase V8 de la souche V8 de Staphylococcus aureus qui clive la liaison peptidique au niveau du groupe carboxylique du résidu Glu ;
- les enzymes non-spécifiques comme la thermolysine provenant de la bactérie Bacillus thermoproteolyticus qui hydrolyse la liaison peptidique du groupe NH2 des acides aminés hydrophobes (Xaa-Leu, Xaa-Ile, Xaa-Phe), la subtilisine et la pronase qui sont des protéases bactériennes qui hydrolysent pratiquement toutes les liaisons et peuvent transformer les protéines en oligopeptides dans des conditions de réaction contrôlées (concentration en enzyme et durée de réaction).

Plusieurs protéases peuvent être utilisées de façon simultanée, si leurs modes d'action sont compatibles, ou elles peuvent être utilisées de façon successive. Dans le cadre de l'invention, la digestion de l'échantillon est, de préférence, réalisée par action d'une enzyme protéase ayant une bonne sélectivité de sites de clivage, par exemple la trypsine.

L'obtention de peptides à l'aide d'un réactif chimique ou d'une protéase, peut être obtenue par simple réaction en solution. Elle peut également être mise en oeuvre avec un four à micro-ondes [1], ou sous pression [16], ou bien encore avec un dispositif à ultrasons [17]. Dans ces trois derniers cas, le protocole peut être beaucoup plus rapide.

Parmi les peptides obtenus par digestion enzymatique (protéolyse enzymatique), les peptides spécifiques de la protéine sont nommés peptides protéotypiques. Ce derniers sont aisément détectés par spectrométrie de masse ou autres techniques analytiques appropriées et adaptées à la détection de tels peptides protéotypiques. Ceci représente un avantage additionnel par rapport à une protéolyse chimique, obtenue au moyen de traitements physico-chimiques.

De préférence, l'agent dénaturant est un agent réducteur de thiols, de préférence sélectionné parmi le 2-mercaptoéthanol , le tris(2-carboxyethyl)phosphine (TCEP), le dithioerythritol (DTE), la tributylphosphine et le dithiothreitol (DTT), avantageusement ledit agent réducteur de thiols est le tris(2-carboxyethyl)phosphine ou le dithiothreitol.

De préférence, l'agent alkylant est sélectionné dans le groupe constitué par la N-éthylmaléimide, l'iodoacétamide et la M-biotine, préférablement ledit agent alkylant est l'iodoacétamide (IAA).

En utilisant les paramètres du procédé selon la présente invention, l'on parvient à diminuer de manière drastique la durée minimale de l'étape de protéolyse enzymatique d), jusqu'alors facteur limitant. En effet, la durée minimale de cette étape de protéolyse enzymatique dans le cadre du procédé d'obtention de peptides selon la présente invention est d'environ 15 minutes (de préférence cette durée minimale est de 5 minutes). La réduction de la durée minimale requise pour obtenir les peptides à l'issue de l'étape de protéolyse enzymatique permet de diminuer, de manière très significative, la durée totale du procédé d'obtention de peptides selon la présente invention, puisque ce dernier peut être réalisé en à peine une demi-heure environ.

D'une manière générale, la durée totale du procédé d'obtention de peptides selon l'invention est inférieure à 1 heure et 45 minutes, de préférence inférieure à 1 heure, avantageusement inférieure à 45 minutes et, de manière optimale, d'environ 30 minutes.

Le gain de temps significatif ainsi obtenu est tout à fait important notamment eu égard aux exigences inhérentes aux domaines clinique et/ou pharmaceutique. Qui plus est, le procédé d'obtention de peptides selon la présente invention est parfaitement adapté aux échantillons complexes (plasma, urine, liquide céphalorachidien etc.) qui sont susceptibles de nécessiter des stratégies de fractionnement ou d'épuisement additionnelles afin d'isoler les protéines cibles préalablement à la mise en oeuvre du procédé d'obtention de peptides. Ainsi, et même en incluant le temps nécessaire à la réalisation de telles stratégies additionnelles de fractionnement ou d'épuisement, le temps de traitement de tels échantillons complexes en appliquant le procédé selon l'invention demeure très avantageux en comparaison avec les procédés de l'art antérieur.

Le traitement préalable de purification d'échantillon de cellules procaryotes et/ou eucaryotes, avant l'étape de lyse a) est connu de l'homme du métier et pourra notamment mettre en oeuvre des techniques de centrifugation, de filtration, d'électrophorèse ou de chromatographie. Ces techniques séparatives peuvent être utilisées seules ou combinées entre elles pour obtenir une séparation multidimensionnelle. Par exemple, une chromatographie multidimensionnelle peut être utilisée en associant une séparation par chromatographie d'échange d'ions à une chromatographie en phase inverse, comme décrit par T. Fortin et al. [2], ou H. Keshishian et al. [3]. Dans ces publications, le milieu chromatographique peut être en colonne ou en cartouche (extraction en phase solide). La fraction électrophorétique ou chromatographique (ou le temps de rétention en chromatographie mono ou multidimensionnelle) des peptides protéotypiques est caractéristique de chaque peptide et la mise en oeuvre de ces techniques permet donc de sélectionner le ou les peptides protéotypiques à doser. Un tel fractionnement des peptides générés permet d'accroître la spécificité du dosage ultérieur par spectrométrie de masse.

Une alternative aux techniques d'électrophorèse ou de chromatographie, pour le fractionnement des peptides, consiste à purifier spécifiquement les N-glycopeptides ([1] et demande de brevet WO 2008/066629). Néanmoins, une telle purification ne permet que la quantification des peptides ayant subi une modification post-traductionnelle de type N-glycosylation. Or toutes les protéines ne sont pas glycosylées, ce qui limite donc son utilisation.

En termes de traitement préalable de purification de l'échantillon de cellules procaryotes et/ou eucaryotes, le procédé selon l'invention comprend, de préférence, avant l'étape a), les étapes additionnelles suivantes :
a') centrifugation des micro-organismes à une vitesse de rotation comprise entre 3500 et 4500 rpm, de préférence de l'ordre de 4000 rpm, durant une période de temps comprise entre 4 minutes et 6 minutes, avantageusement de 6 minutes, à une température comprise entre 15°C et 25°C, préférablement d'environ 20°C,
a") Elimination du surnageant et récupération du culot contenant les micro-organismes,
a'") Reprise dudit culot dans un solvant.

Avantageusement, le solvant utilisé dans le cadre de la présente invention est sélectionné parmi une solution d'acétonitrile et une solution aqueuse comprenant un tampon de pH tel que des ions carbonates, avantageusement ledit solvant est une solution aqueuse comprenant des ions carbonate, telle qu'une solution de bicarbonate d'ammonium.

Comme pour l'étape de traitement préalable de purification d'échantillon susvisée, le procédé peut comprendre, le cas échéant, postérieurement à l'étape de récupération des peptides e), des étapes de concentration desdits peptides. A titre d'exemple, l'étape de récupération des peptides e) peut être suivie des étapes suivantes :
e1) Centrifugation à une force centrifuge relative comprise entre 13000g et 15000g, avantageusement d'environ 14000g, pendant une durée comprise entre 25 minutes et 35 minutes, avantageusement d'environ 30 minutes, à une température comprise entre 2°C et 6°C, avantageusement d'environ 4°C,
e2) récupération de tout ou partie du surnageant comportant les peptides.

Selon la présente invention, au moins les étapes de lyse a) et dénaturation b) sont effectuées conjointement/simultanément.

Selon un premier aspect de l'invention, les étapes a) et b) sont effectuées conjointement/simultanément. Ainsi, et contrairement aux procédés de l'art antérieur, le temps de lyse est mis à profit pour dénaturer les protéines. Ceci permet de raccourcir la durée du procédé dans son ensemble et d'en faciliter l'automatisation.

Dans le cadre de ce premier aspect de l'invention, l'étape d'alkylation des protéines dénaturées c) est effectuée à une température connue de l'homme du métier pour être apte et à même de permettre la réalisation du traitement d'alkylation desdites protéines. Cette étape d'alkylation des protéines c) est réalisée
préférentiellement à l'abri de la lumière et à une température comprise entre 10°C et 60°C, préférablement entre 15°C et 25°C, avantageusement à température ambiante.

Toujours dans ce premier aspect de l'invention, l'étape de protéolyse enzymatique d), quant à elle, est effectuée à une température appropriée, à savoir également connue de l'homme du métier pour permettre la digestion enzymatique des protéines (protéolyse enzymatique). Avantageusement, cette étape de protéolyse d) est effectuée à une température comprise entre 30°C et 60°C, de préférence entre 37°C et 55°C, avantageusement environ 50°C.

Dans ce premier aspect de l'invention, la durée des étapes conjointes (simultanées) de lyse a) et de dénaturation b) est comprise entre 3 minutes et 7 minutes, de préférence entre 4 minutes et 6 minutes, avantageusement ladite durée est d'environ 5 minutes.

En outre, et toujours dans ce premier aspect de l'invention, la durée de l'étape d'alkylation des protéines dénaturées c) est également comprise entre 3 minutes et 7 minutes, de préférence entre 4 minutes et 6 minutes, avantageusement ladite durée est d'environ 5 minutes.

Toujours selon ce premier aspect de l'invention, le procédé comprend, après l'étape d'alkylation des protéines dénaturées c), l'étape suivante :
c1) dosage des protéines obtenues à ladite étape c),
et dans lequel l'enzyme protéolytique est ajoutée lors de l'étape de digestion enzymatique d) dans un ratio en poids (w/w) par rapport au poids des protéines dosées à l'étape c1) compris entre 1/5 et 1/15, de préférence entre 1/8 et 1/12, avantageusement d'environ 1/10.

Selon un deuxième aspect de l'invention, au moins les étapes a) - c) du procédé selon l'invention sont effectuées conjointement (a) + b) + c)).

Selon un mode de réalisation particulier de ce deuxième aspect de l'invention, les étapes a) - c) (lyse, dénaturation et alkylation) sont effectuées conjointement (simultanément).

Selon un troisième aspect de la présente invention - particulièrement préféré - les étapes a) - d) sont effectuées conjointement (simultanément).

Selon ce troisième aspect de la présente invention - particulièrement préféré - les étapes a) - d) du procédé selon la présente invention sont effectuées conjointement (a) + b) + c) + d)).

Plus précisément, toutes les étapes a) - d) sont effectuées dans un même contenant (par exemple un tube eppendorf) sans nécessiter l'ajout de réactifs ni de manipulation entre lesdites étapes. Ce procédé est donc aisément automatisable, ce qui représente un avantage majeur dans une optique de réduction des coûts, de limitation des risques liés à une manipulation erronée, ainsi que de réduction des volumes à utiliser (liste non limitative).

Dans le cadre des procédés selon les deuxième et troisième aspects de la présente invention, il est nécessaire de s'assurer de la compatibilité des agents utilisés aux différentes étapes b), c) et d), et plus particulièrement en ce qui concerne la compatibilité de l'agent dénaturant et de l'agent alkylant, ce dernier ayant une tendance naturelle à alkyler l'agent dénaturant introduit à l'étape b).

De préférence, selon ces deuxième et troisième aspects de l'invention :
- l'agent dénaturant est un agent réducteur de thiols sélectionné parmi le tris(2-carboxyethyl)phosphine et le dithiothreitol, avantageusement ledit agent réducteur de thiols est le tris(2-carboxyethyl)phosphine, et
- l'agent alkylant est l'iodoacétamide.

En effet, la demanderesse a découvert, de façon surprenante, que l'utilisation du couple TCEP/iodoacétamide (IAA) permettait d'obtenir de très bons rendements concernant le procédé selon la présente invention.

Un autre objet de la présente invention concerne un procédé d'analyse de peptides de cellules procaryotes et/ou eucaryotes comprenant les étapes suivantes :
i) Obtention des peptides à partir desdites cellules procaryotes et/ou eucaryotes en utilisant le procédé selon l'invention,
ii) Analyse des peptides ainsi obtenus, ladite analyse étant effectuée à l'aide d'un moyen d'analyse de type spectrométrie de masse.

A titre d'exemple, ce procédé d'analyse de peptides peut être, dans le domaine de la microbiologie, un procédé de caractérisation d'au moins un micro-organisme issu d'un échantillon, comprenant, par exemple, l'identification dudit microorganisme et la détermination des propriétés de typage, résistance potentielle à au moins un antimicrobien et facteur de virulence concernant ledit microorganisme.

Un autre exemple concerne la recherche de biomarqueurs, notamment à partir d'échantillons biologiques complexes tels que le plasma, l'urine, le liquide céphalorachidien, etc.

La présente invention concerne également un kit d'obtention de peptides à partir de cellules procaryotes et/ou eucaryotes, ledit kit étant spécifiquement adapté à la mise en oeuvre du procédé selon la présente invention, ledit kit comprenant :
- un nécessaire de lyse permettant d'effectuer la lyse à faible concentration en agent(s) chaotropique(s) des cellules procaryotes et/ou eucaryotes, de préférence un nécessaire de lyse par sonication, ledit nécessaire de lyse permettant en outre d'effectuer la lyse sous une pression inférieure à 100 bars, de préférence inférieure à 50 bars, avantageusement inférieure à 10 bars, et de manière particulièrement préférée sous pression atmosphérique,
- une première solution comprenant au moins un agent dénaturant, de préférence sélectionné parmi le 2-mercaptoéthanol, le tris(2-carboxyethyl)phosphine, le dithioerythritol, la tributylphosphine et le dithiothreitol, avantageusement ledit agent dénaturant est le tris(2-carboxyethyl)phosphine ou le dithiothreitol,

- une deuxième solution comprenant au moins un agent alkylant, de préférence sélectionné dans le groupe constitué par la N-éthylmaléimide, l'iodoacétamide et la M-biotine, préférablement ledit agent alkylant est l'iodoacétamide,
- une troisième solution comprenant au moins une enzyme protéolytique, telle qu'une protéase à sérine, préférablement sélectionnée dans le groupe consistant en la trypsine, la chymotrypsine et l'élastase, avantageusement ladite enzyme protéolytique est la trypsine.

Ce kit comprend, de manière optionnelle, des instructions d'utilisation définissant ses modalités d'utilisation.

Selon un mode de réalisation préféré, ledit kit comprend :
- un nécessaire de lyse par sonication,
- une première solution comprenant un agent dénaturant sélectionné parmi le 2-mercaptoéthanol, le tris(2-carboxyethyl)phosphine, le dithioerythritol, la tributylphosphine et le dithiothreitol, avantageusement ledit agent dénaturant est le tris(2-carboxyethyl)phosphine ou le dithiothreitol,
- une deuxième solution comprenant un agent alkylant consistant en l'iodoacétamide,
- une troisième solution comprenant une enzyme protéolytique consistant en la trypsine.

De préférence, le nécessaire de lyse et les première, deuxième et troisième solutions sont adaptées et destinées à être utilisées conjointement (simultanément), notamment dans le cadre du procédé selon le troisième aspect de la présente invention, tel qu'indiqué précédemment.

Encore un autre objet de la présente invention concerne l'utilisation d'un kit selon l'invention pour la mise en oeuvre du procédé d'obtention de peptides susvisé et/ou du procédé d'analyse de peptides également mentionné ci-dessus.

### Brève description des dessins

L'invention, sa fonctionnalité, ses applications ainsi que ses avantages seront mieux appréhendés à la lecture de la présente description, faite en référence aux figures suivantes, dans lesquelles :
- La figure 1 représente de façon schématique, un procédé de préparation d'un échantillon de micro-organismes selon l'art antérieur (P0), ce procédé comprenant notamment une étape de lyse à forte concentration saline (guanidine 6M - agent chaotropique) et une longue étape de digestion trypsique,
- La figure 2 est un schéma montrant les différentes étapes d'un procédé selon un premier aspect de la présente invention (P1),
- La figure 3 représente les données relatives à l'établissement d'une gamme d'étalonnage de sérum-albumine bovine (BSA), permettant ainsi d'obtenir une correspondance entre la densité optique (DO) mesurée à 595 nm et la concentration en protéines (mg/ml), dans le cadre du susdit premier aspect de la présente invention (P1)
- Les figures 4, 5 et 6 présentent les résultats obtenus après mise en oeuvre du procédé P1, respectivement sur chacun des trois micro-organismes testés, à savoir Escherichia coli EC5, Staphylococcus epidermidis SE9 et Candida albicans CA16,
- La figure 7 a trait à l'évaluation des conséquences éventuelles de l'absence d'étape de préchauffage de la sonde à ultrasons dans le cadre du susdit procédé P1,
- La figure 8 est un schéma montrant les différentes étapes d'un procédé selon un troisième aspect de la présente invention (P2), particulièrement avantageux, dans lequel l'agent dénaturant utilisé est le TCEP (« P2-TCEP »),
- La figure 9 est un graphique représentant les résultats des analyses LC-ESI-MS effectuées à l'issue du protocole P2-TCEP sur chacun des trois micro-organismes susvisés, à savoir Escherichia coli EC5, Staphylococcus epidermidis SE9 et Candida albicans CA16,
- La figure 10 est un schéma montrant les différentes étapes du procédé selon le troisième aspect de la présente invention (P2), particulièrement avantageux, dans lequel l'agent dénaturant utilisé est le DTT (P2-DTT),- La figure 11 est un graphique représentant les résultats des analyses LC-ESI-MS effectuées à l'issue du protocole P2-DTT sur chacun des trois micro-organismes susvisés, à savoir Escherichia coli EC5, Staphylococcus epidermidis SE9 et Candida albicans CA16.

### Description détaillée de l'invention

Les exemples présentés ci-après permettent de mieux illustrer la présente invention. Toutefois, ces exemples ne doivent en aucun cas être regardés comme limitant la portée de ladite invention d'une quelconque manière que ce soit.

### Exemple 1 : Protocole P0

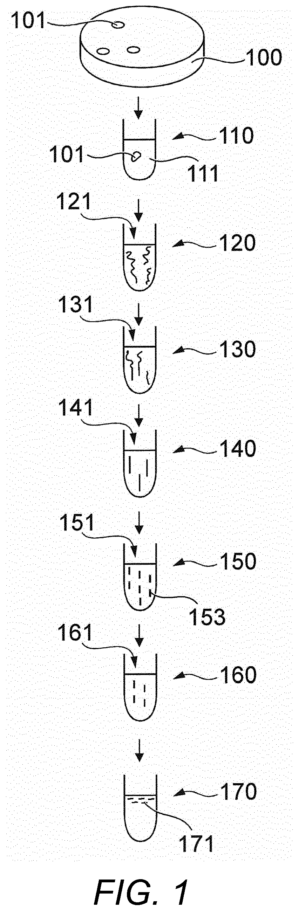

Le protocole P0 est un protocole de préparation d'échantillon traditionnellement utilisé dans l'art antérieur afin d'obtenir des peptides à partir de micro-organismes. Ce procédé P0 comprend notamment une étape de lyse à forte concentration en sel (guanidine 6M : agent chaotropique) ainsi qu'une longue étape de digestion trypsique. P0 est utilisé ci-après comme référence afin d'évaluer la qualité/l'efficacité des protocoles d'obtention de peptides selon un premier aspect de l'invention (protocole P1) Le susdit protocole P0 - décrit ci-après en référence à la figure 1 - dure approximativement 24 heures et comprend les étapes suivantes :
- mise en suspension d'une à trois colonies d'un micro-organisme 101 (tel qu'une bactérie de type E.coli) cultivé sur une boîte de Petri 100 dans un tube comprenant une solution 111 composée de 100 µL d'hydrochlorure de guanidine 6M, 50 mM Tris-HCl, pH 8, et laisser la réaction de lyse 110 se produire durant une période de 10 minutes environ;
- introduire, lors de l'étape de dénaturation/réduction 120, une solution 121 comprenant 3,5 µL de DTT à une concentration de 150 mM, de façon à obtenir une solution finale de DTT à 5 mM ; cette étape de dénaturation/réduction étant réalisée en 30 minutes environ;
- effectuer ensuite l'étape d'alkylation 130 en introduisant une solution 131 d'IAA à une concentration de 150 mM et laisser la réaction d'alkylation 130 se produire durant 45 minutes environ, afin d'opérer un blocage (protection) des fonctions thiols sur les protéines dénaturées ;
- en préalable à l'étape de digestion trypsique 150, effectuer une étape de dilution 140 en ajoutant 500 µL d'une solution 141 comprenant 50 mM de NH₄HCO₃, afin de diminuer la forte concentration en agent chaotropique (guanidine 6M) pour ne pas nuire à l'activité enzymatique de la trypsine lors de l'étape ultérieure de digestion trypsique 150 ;
- réaliser cette étape de digestion trypsique 150 en introduisant 0,5 à 3 µL d'une solution 151 de trypsine dosée à 1 µg/µL; laisser la digestion enzymatique se produire durant 6 à 20 heures (durée permettant d'obtenir les peptides 153);
- stopper à l'étape 160 la susdite digestion trypsique 150 en ajoutant 2 µL d'acide formique 161 ; puis
- séparer les peptides 171 ainsi obtenus, par exemple par centrifugation pour éliminer les espèces non solubles qui pourraient gêner les étapes analytiques ultérieures et prélever ces derniers en vue de réaliser, le cas échéant, des étapes ultérieures d'analyse(s).

### Exemple 2 : Protocole P1

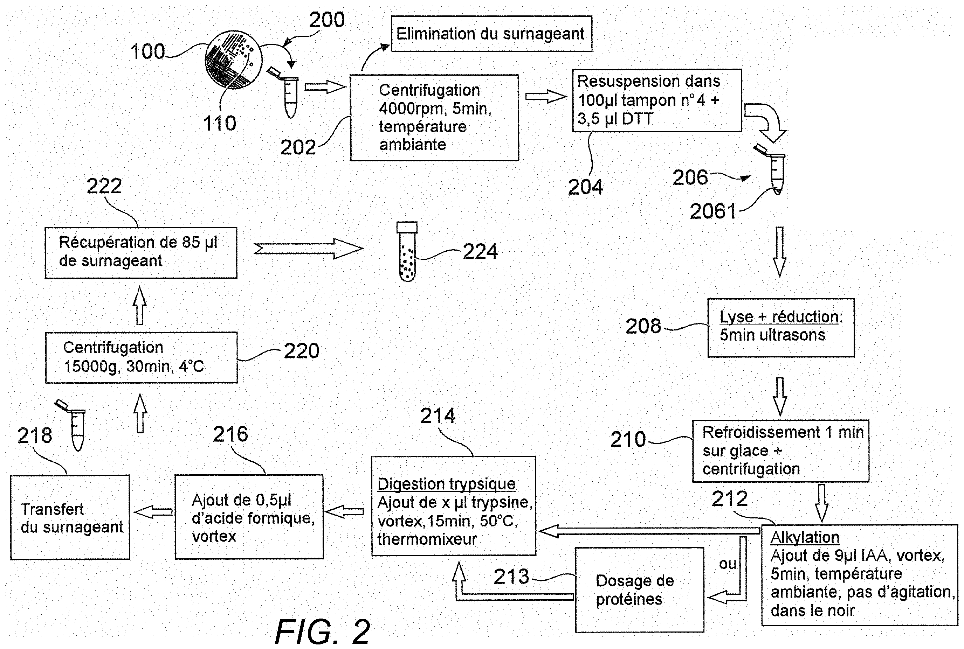

Un procédé selon un premier aspect de l'invention - dénommé « protocole P1 » ou « procédé P1 » - est décrit ci-après, en référence à la figure 2.

Ce protocole P1 permet une préparation très rapide de l'échantillon préalablement aux étapes d'analyse ultérieures, réalisées, par exemple, par spectrométrie de masse. La durée de l'ensemble des étapes du protocole P1 est d'environ 30 minutes. Il comprend notamment les étapes essentielles suivantes :
- lyse et réduction 208 (effectuées conjointement/simultanément), en présence de DTT, 5min sous ultrasons ,
- alkylation 212 : en présence d'IAA, 5min à température ambiante et sans agitation, et
- protéolyse enzymatique (également dénommée digestion enzymatique) 214 : en présence de trypsine, 15 min à 50 °C.

Comme indiqué précédemment, le protocole P1 est effectué approximativement en 30 minutes et donne des résultats semblables (analyses MRM) à ceux obtenus par le protocole P0 (cf. Exemple 1 - durée de traitement : 24h), sur chacun des trois micro-organismes testés, à savoir : Escherichia coli EC5 (Gram- ; désigné ci-après « EC5 »), Staphylococcus epidermidis SE9 (Gram+ ; désigné ci-après « SE9 ») et Candida albicans CA16 (levure ; désigné ci-après « CA16 »).

### Matériel et méthode

### 2.1. Produits utilisés

- BSA / Sigma/référence A9085-5G/n° lot : 097K1513
- Réactif Bradford : "Quick Start Bradford Dye Reagent 1X"/Bio-Rad/référence 500-0205/control 210006065
- Acide formique /Fluka/référence : 06450
- Iodoacétamide, (IAA)/Sigma/I6125-5G/lot 099K5300/MM=184,96
- DL-1,4-Dithiothreitol 99%, (DTT)/Acros/165680010/lotA0269816/MM=154,24
- Ammonium bicarbonate/Sigma/A6141-500g/lot n°117K0039/MM=79,06
- Hydroxyde d'ammonium, NH₄OH/ammoniaque 28%/référence: 21190292/MM=17,03g
- Trypsine / Promega/référence V511 (stockage à -20°C)
- « Suspension médium » (eau stérile) bioMérieux (réf. : 70640)
- Souche Escherichia coli, n° ATCC : 11775T
- Souche Staphylococcus epidermidis, n°ATCC : 14990
- Souche Candida albicans, n°ATCC : 18804

### 2.2. Préparation des solutions tampons

- Tampon n°4 : bicarbonate d'ammonium 50mM pH8/stockage 1 mois à +4°C Pour 50ml tampon : 197,6mg de bicarbonate qsp 50ml H₂O/pH=7,9 ; ajouter environ 10µl de NH₄OH pour obtenir pH=8
- Tampon n°5 : DTT 150mM/à préparer extemporanément Pour 1ml de tampon : 23,1mg de DTT qsp 1ml tampon bicarbonate n°4
- Tampon n°6 : IAA 150mM/à préparer extemporanément Pour 1 ml tampon : 27,7mg d'IAA qsp 1ml tampon bicarbonate n°4

### 2.3. Matériel

- Centrifugeuse "APPLI 24"/Prolabo
- Tubes 1,5ml ; « Safe Lock » Eppendorf, réf : 0030120.086
- Centrifugeuse "paillasse"/Eppendorf/ref 5415C
- Thermomixer "comfort"/Eppendorf
- Lecteur microplaque (595nm) + microplaques+modules
- Spectrophotomètre UVIKON + cuves quartz 80µl
- Boites culture BMX : COS (réf. :43041) et SDA (réf. : 43555)
- Densichek Plus bioMérieux : référence : 21250
- Sonde à ultrasons « Hielscher » ; réf : PN-66-NNN
- Billes de lyse 0.1mm (petit diamètre) : « Zirconia/Silica beads »/Roth/N033.1
- Billes de lyse 1 mm (grand diamètre) : « Silibeads typ 1/1,3mm/réf: 4504/VWR
- Cavaliers /Dutscher/référence : 011870A

### 2.4. Protocole

### 2.4.1. Etapes 200, 202, 204, 206, 208 et 210

En premier lieu, l'on prépare les solutions tampons n°5 (DTT 150mM) et n°6 (IAA 150mM).

L'on pèse, à l'aide d'une spatule « cuillère », 50mg de billes 0,1mm de diamètre (billes de petit diamètre) et 50mg de billes 1mm de diamètre (billes de grand diamètre) que l'on introduit dans le tube n°1. Le mélange des billes de petit diamètre et grand diamètre est représenté sur la figure 2 par la référence numérique 2061.

L'on prélève une à trois colonies 110 à partir d'une boîte de Petri 100, que l'on met en suspension dans l'eau à l'étape 200. La concentration en bactéries de la suspension ainsi obtenue est estimée par les méthodes classiques de mesure de turbidité (mesure de l'absorption à 550nm). Un volume de suspension correspondant à 1.10⁸ CFU est prélevé puis centrifugé, à l'étape 202, à 4000rpm durant 5 min, à température ambiante. A l'issue de cette étape de centrifugation 202, le culot est repris (EC5, CA16 et SE9) à l'étape 204 dans 100µL de la solution tampon n°4 (tube n°2), puis l'on ajoute, toujours à l'étape 204, 3,5 µl de DTT 150mM (tampon n°5) pour obtenir une concentration finale de 5mM en DTT au sein du tube n°2. L'on vortexe 2 secondes (puissance maximale) pour homogénéiser le contenu de ce tube n°2 et l'on pipette le mélange afin de le transférer dans le tube n°1, à l'étape 206, contenant le mélange de billes de « petit diamètre » et « grand diamètre » 2061. Le tube n°2 est éliminé.

Un cavalier est placé sur le tube n°1 pour éviter qu'il ne s'ouvre lors de la lyse par sonication.

Le tube n°1 est ensuite introduit dans un des orifices de la sonde à ultrasons Hielscher (les 6 orifices à l'extrémité de la sonde sont supposés être identiques selon le fournisseur), puis l'on décompte 5min pour effectuer la lyse/réduction 208 (réglages Amplitude 100/Cycle 1) ; la température du mélange, dans le tube, atteint 95°C environ.

Le tube n°1 est enlevé de l'orifice de la sonde à l'aide d'un « levier » en tenant le tube par le cavalier, puis ce tube n°1 est refroidi, à l'étape 210, en le stockant 1 minute dans de la glace afin de ramener la température du mélange du tube à température ambiante.

L'on centrifuge ensuite brièvement ce tube n°1 à l'aide d'une centrifugeuse de paillasse (en fin d'étape 210), afin de récupérer le liquide présent dans le bouchon et sur les parois.

### 2.4.2. Etape d'alkylation 212 (blocage des ponts disulfures des protéines par méthylation avec l'IAA)

9µL de solution d'IAA à 150mM (tampon n°6) sont ajoutés au sein du tube n°1 à l'étape 212 pour obtenir une molarité finale de 12,5mM, ledit tube n°1 étant ensuite vortexé 2 secondes (puissance maximale) pour homogénéisation.

On laisse la réaction d'alkylation 212 se produire durant 5 min à température ambiante, à l'abri de la lumière.

A ce stade là, deux options sont possibles, à savoir :
- l'on poursuit le protocole P1 avec l'étape de digestion trypsique 214 (cf. section 2.4.4. infra), en utilisant une quantité prédéfinie de trypsine, ou
- l'on effectue, avant cette étape de digestion trypsique 214 (également dénommée protéolyse trypsique), un dosage des protéines 213 (cf. 2.4.3. infra), à réaliser au sein d'un tube n°3 qui ne sert qu'à mettre en oeuvre le dosage des protéines 213, afin d'évaluer la quantité de trypsine à ajouter lors de l'étape de digestion trypsique 214 en fonction de la concentration en protéines dosée à l'issue de l'étape d'alkylation 212.

### 2.4.3. Dosage des protéines 213 (selon la méthode de Bradford)

Le dosage des protéines s'effectue sur le surnageant du lysat centrifugé 5 minutes à 14000rpm. Il est donc nécessaire, dans un premier temps, de vortexer le tube n°1 (homogénéisation du lysat), puis de pipeter le lysat dudit tube n°1 (une partie des billes de 0,1mm est souvent récupérée), de transférer ce lysat dans un tube (tube n°3) et de centrifuger ce tube n°3 à 14000rpm durant 5 minutes. Le dosage s'effectue alors sur le surnageant. Le tube n°3 comprenant les billes restantes est éliminé.

La quantité de protéines est évaluée en fonction d'une gamme d'étalonnage de BSA diluée en tampon carbonate et dosée en parallèle du lysat centrifugé (gamme élaborée à partir d'une solution BSA à 1mg/ml puis préparation de solutions à 0,1/0,2/0,3/0,4/0,6/0,8 et 1mg/ml).

Le dosage est effectué sur 4µl du surnageant du lysat centrifugé et 4µl de chacune des solutions de la gamme, dans 200µl réactif Bradford, en microplaque, avec une lecture de la densité optique (DO) à 595nm.

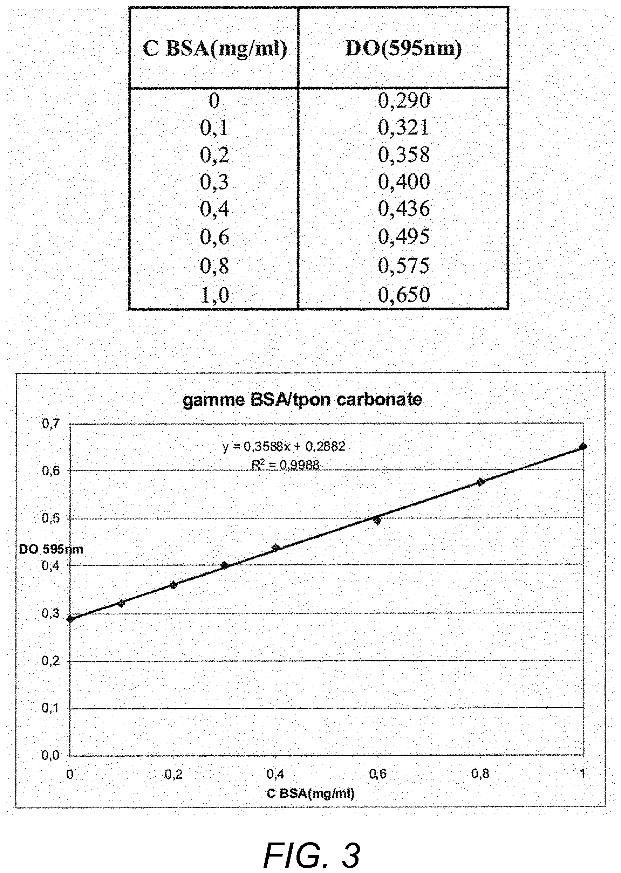

Un Exemple de gamme d'étalonnage est représenté en figure 3.

### 2.4.4. Digestion trypsique des protéines 214

Le thermomixer est préchauffé, au préalable, pendant 15 minutes à 50°C.

L'on utilise une solution de trypsine reprise extemporanément : flacon de 20µg de trypsine Proméga dans 20 µl de la solution de reprise contenue dans le kit (concentration finale 1µg/µl).

Comme cela est connu de l'homme du métier, l'activité maximale de la trypsine s'effectue à un pH de 7/9.

Si un dosage des protéines a été effectué à l'étape 213, la trypsine 1µg/µl est ajoutée à l'étape 214 avec un ratio en poids (w/w) protéase/protéine de 1/10, soit 1µl (=1µg) pour 10µg de protéines.

En revanche, si un tel dosage des protéines 213 n'a pas été effectué entre l'étape d'alkylation 212 et celle de digestion trypsique 214, la trypsine 1µg/µl est introduite dans le tube n°1, à l'étape 214, à une quantité standard de 10 µl. En effet la Demanderesse a déterminé que cette quantité standard de trypsine permettait d'obtenir une digestion trypsique satisfaisante dans l'ensemble des conditions de quantités de micro-organismes pertinentes pour ce qui concerne les applications d'analyse peptidique souhaitées.

Toujours à l'étape 214, et postérieurement à l'ajout de trypsine, le mélange est vortexé durant 2 secondes (puissance maximale) pour homogénéisation.

Ensuite, l'on incube ce mélange en thermomixer, 15 minutes à 50°C, 850rpm.

### 2.4.5. Arrêt de la digestion trypsique 216

L'arrêt de la digestion trypsique est effectué par un ajout d'acide formique (qsp pH inférieur à 4) dans le tube n°1 à l'étape 216. En effet, la trypsine devient inactive à un pH inférieur à 4 (ce phénomène est réversible et la trypsine redevient active à un pH supérieur ou égal à 4).

Le pH de l'échantillon était d'environ 8 (pH vérifié au pH mètre/sonde spéciale micro-tubes) avant ajout de 0,5µl d'acide formique. Il est d'environ 3 après ajout de ce dernier à l'étape 216. Le mélange est ensuite vortexé 2 secondes (puissance maximale) pour homogénéisation.

### 2.4.6. Etapes 218, 220, 222 et 224

Les peptides résultant de la digestion trypsique se trouvent dans le surnageant.

Le surnageant du tube n°1 est pipeté (une partie des billes de 0,1mm est souvent récupérée) et transféré dans un autre tube (tube n°4) à l'étape 218 puis centrifugé, à l'étape 220, durant 30 minutes à 15000g, à une température de 4°C.

85µl de surnageant comportant les peptides sont ensuite récupérés à l'étape 222 et introduits dans un autre tube (tube n°5) à l'étape 224. Ce dernier est stocké au congélateur à -20°C.

Les peptides contenus dans le tube n°5 sont ensuite analysés par MRM afin de déterminer la qualité/l'efficacité du protocole P1 (étape de validation des résultats). Les résultats de ces analyses sont présentés ci-après.

### 2.5. Validation des résultats

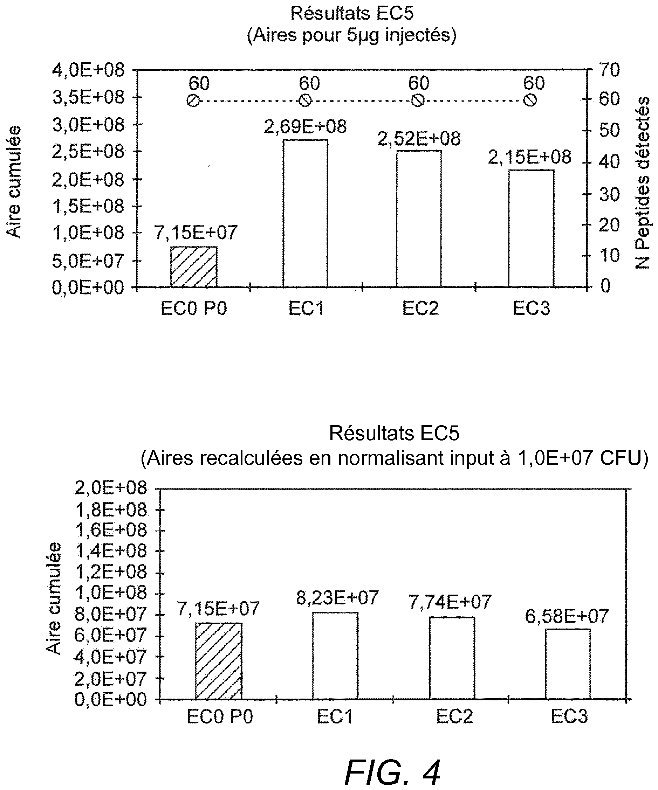

Comme indiqué précédemment, ce protocole d'obtention de peptides P1 a été validé en MRM. Les résultats, pour chacun des trois micro-organismes étudiés (EC5, SE9 et CA 16), sont respectivement présentés en figures 4, 5 et 6.

La figure 4 montre les résultats d'analyse en mode MRM de peptides obtenus avec le protocole P1 à partir de 1^{e}8 CFU de E.coli EC5. L'expérience a été reproduite trois fois (colonnes EC1, EC2, EC3) et comparées aux résultats obtenus à partir du même inoculum en utilisant le protocole P0 (barre hachurée). Les barres représentent la somme des aires sous pic des peptides correctement détectables dans le chromatogramme de masse obtenu en analyse LC-ESI-MS en mode MRM. Cette aire totale représente l'intensité des signaux obtenus et est liée à la concentration des peptides correspondants dans la solution analysée. Plus cette aire est grande, plus cette concentration est élevée. Les figures indiquent également (points reliés par des lignes) le nombre de peptides correctement détectés, sur un total possible de 60 pour les réglages de l'analyse qui ont été choisis.

Le premier graphique (partie haute de la figure 4) représente les données obtenues en normalisant la quantité de protéines présente dans l'échantillon analysé en LC-ESI-MS. Le second graphique (partie basse de la figure 4) représente les données obtenues en modifiant le volume de produit de réaction P1 de façon analyser l'équivalent de 1e7 CFU initiales. Dans ce cas, la comparaison des aires sous pics permet de comparer les rendements globaux obtenus sur les différents échantillons.

En conclusion il apparaît que :
- le procédé P1 est reproductible sur la somme des aires cumulées sous les pics des peptides détectés, ce qui signifie que les intensités de ces pics sont globalement reproductibles et suggère que les rendements des différentes étapes du protocole sont reproductibles,
- ce procédé est équivalent au procédé de référence P0 (présenté dans l'Exemple 1) en termes du nombre de peptides détectés pour ce qui concerne EC5,
- ce procédé P1 permet une digestion par la trypsine plus efficace que dans les conditions du protocole P0, comme en témoigne le fait que l'analyse de masses protéiques équivalentes avant digestion se traduit par des intensités de signaux des peptides supérieures pour P1 que pour P0.
- en revanche, à quantité de bactéries égale en début de protocole, les intensités de détection des peptides sont équivalentes pour les deux protocoles, ce qui semblerait suggérer que les étapes avant digestion du protocole P1 sont moins efficaces que dans le protocole P0.

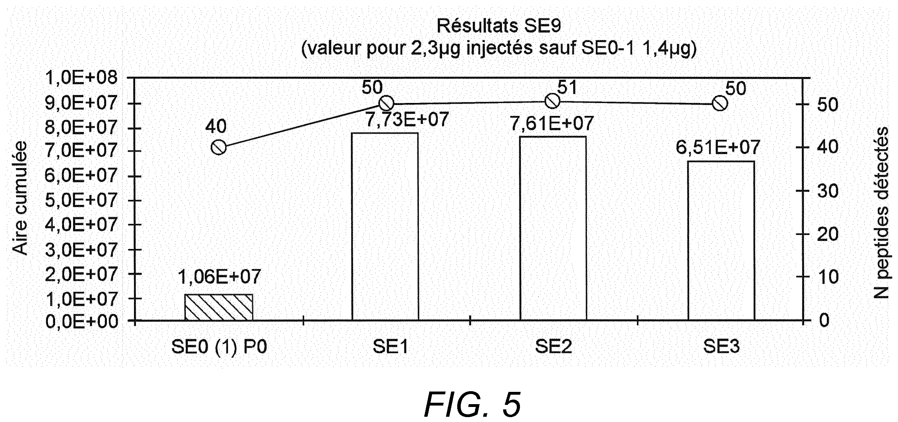

La figure 5 est l'équivalent du premier graphique de la figure 4 (en partie haute) dans le cas où le micro-organisme étudié est S.epidermidis SE9. D'après cette figure 5, il apparaît que les conclusions tirées ci-dessus dans le cas de E*.coli* EC5 sont également valables dans le cas d'autres espèces de bactéries - notamment S.*epidermidis* SE9. Cela est d'autant plus vrai que la masse équivalente de protéines injectées est plus faible dans la condition P0 que dans les conditions P1.

En conclusion, et malgré le fait que la quantité injectée soit plus faible pour P0, le procédé P1 peut être regardé comme à tout le moins équivalent à P0 concernant la détection de peptides de SE9.

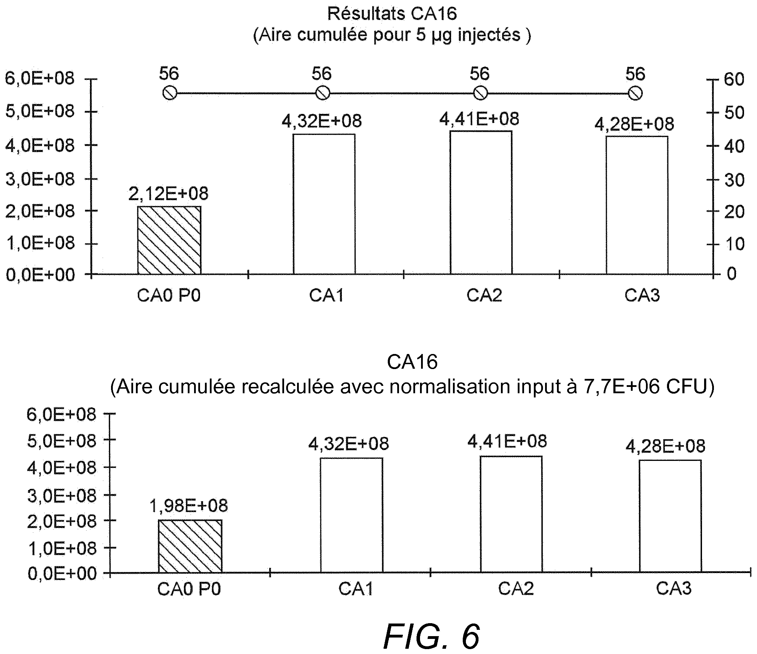

La figure 6, quant à elle, montre qu'en ce qui concerne la détection de peptides de CA16:
- le procédé P1 est reproductible sur la somme des aires et,
- ce procédé est supérieur au procédé de référence P0.

### 2.6. Influence de l'étape de préchauffage de la sonde à ultrasons

Le protocole P1 détaillé ci-dessus comprend une étape de préchauffage de la sonde à ultrasons « Hielscher » en laissant cette sonde fonctionner « à vide » dans les conditions utilisées ensuite pour l'étape de lyse du protocole P1 durant 1 heure. Dans ces conditions, la température du bloc vibrant de la sonde est d'environ 95°C. Cette étape de préchauffage étant susceptible de diminuer la durée de vie de la sonde à ultrasons, la demanderesse a cherché à déterminer les conséquences éventuelles d'une omission de la susdite étape de préchauffage de cette sonde à ultrasons dans le cadre du protocole P1. Le protocole P1 ainsi modifié est dénommée P1'.

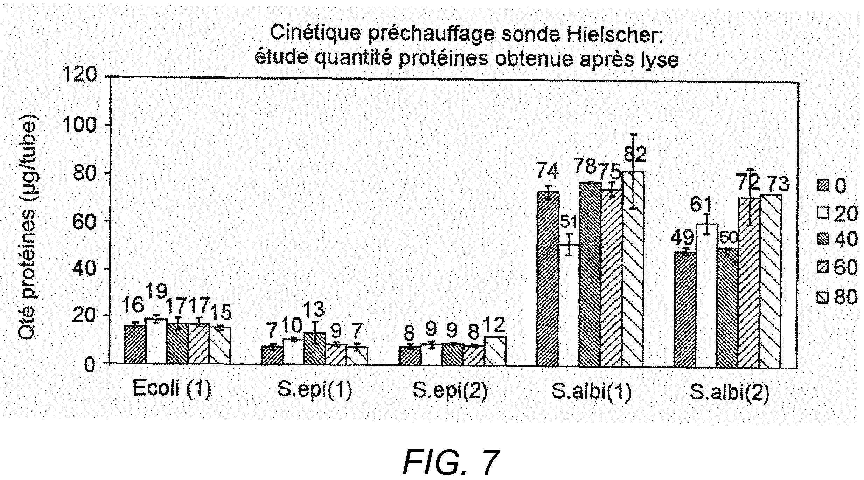

La figure 7 représente les quantités de protéines dosées postérieurement à l'étape de lyse par sonication et réduction 208 (cf. figure 2) après différents temps de fonctionnement de la sonde de sonication (0, 20, 40, 60, 80 minutes, le temps 0 min correspondant à l'utilisation d'une sonde utilisée après un temps d'inactivité suffisant pour que le bloc vibrant se trouve à température ambiante). Les concentrations en protéines ont été déterminées par un test de Bradford et sont reportées sur le graphiques et exprimées en µg pour 1^{e}8 cellules bactériennes.

Ce dosage peut être effectué par la méthode de Bradford ou par toute autre méthode appropriée, connue de l'homme du métier.

Les résultats représentés sur cette figure 7 confirment qu'il n'est pas nécessaire de préchauffer la sonde avant son utilisation pour obtenir une lyse bactérienne de qualité.

En outre, les analyses MRM confirment également que les essais de digestion trypsique des trois souches EC5, SE9 et CA16, sans préchauffage de la sonde à ultrasons utilisée pour effectuer la lyse (protocole P1'), donnent des résultats supérieurs au protocole de référence P0.

Outre les avantages inhérents aux procédés d'obtention de peptides selon la présente invention (cf. ci-dessus), ces procédés permettent de se dispenser de l'étape préalable de préchauffage de la sonde à ultrasons, dans le cadre d'une lyse par sonication. Ceci résulte notamment en une longévité accrue de ladite sonde à ultrasons ainsi qu'en un gain d'énergie.

### Exemple 3 : Protocole P2

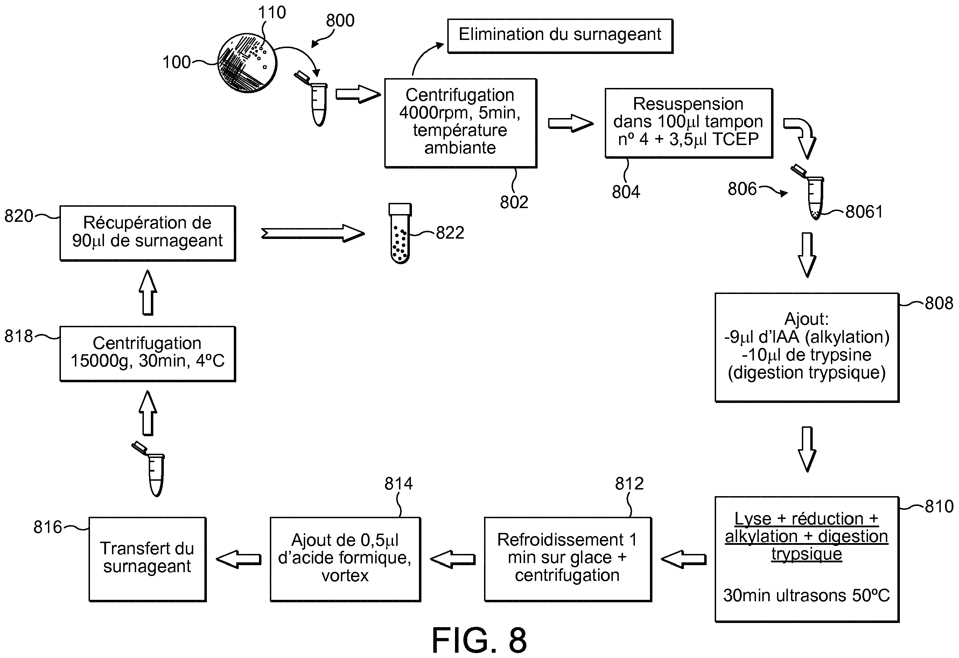

Un troisième aspect de l'invention, dénommé « protocole P2 » (ou « procédé P2 »), est décrit ci-après, en référence à la figure 8. Ce procédé P2 est particulièrement préféré au sens de la présente invention.

Dans cet Exemple 3, le procédé P2 est utilisé pour obtenir des peptides à partir de micro-organismes.

Ce procédé P2 est un protocole d'obtention de peptides à partir de cellules procaryotes et/ou eucaryotes très rapide, pouvant être employé notamment en préalable à des étapes d'analyse de type analyse par spectrométrie de masse.

Tout comme le protocole P1, ce protocole P2 est également effectué en 30 min environ mais combine les étapes de lyse, dénaturation/réduction, alkylation et digestion enzymatique en une seule étape 810. Ces différentes étapes sont en effet effectuées conjointement/simultanément dans un seul et même tube à 50°C, sous ultrasons pendant 30 minutes Ainsi, nul besoin d'effectuer des interventions manuelles entre ces étapes, pas plus que de rajouter des réactifs en cours de procédé. Le protocole P2 est donc facile à automatiser, évite d'éventuelles erreurs de manipulation et permet l'utilisation de volumes plus faibles. En outre, il autorise également l'analyse d'échantillons complexes (urine, plasma, liquide céphalorachidien), tel qu'expliqué précédemment.

Ce protocole P2 donne des résultats semblables (analyses LC-ESI-MS) aux résultats du protocole P1 décrit dans l'exemple 2, sur deux des trois micro-organismes testés : Escherichia Coli (Gram-) et Staphylococcus epidermidis (Gram+) tant en nombre de peptides qu'en aire cumulée.

### Matériel et méthode

### 3.1 Produits utilisés

- Acide formique /Fluka/référence : 06450
- Iodoacétamide, (IAA)/Sigma/I6125-5G/MM=184,96
- Tris(2-carboxyethyl) phosphine (TCEP) hydrochloride solution à 0,5M /Sigma/646547
- Ammonium bicarbonate/Sigma/A6141-500g/MM=79,06
- Hydroxyde d'ammonium,NH₄OH/ammoniaque 28%/référence: 21190292/MM=17,03g
- Trypsine / Promega/référence V511 (stockage à -20°C)
- « Suspension médium » (eau stérile) bioMérieux (réf. : 70640)
- Souche Escherichia coli, n° ATCC : 11775T
- Souche Staphylococcus epidermidis, n°ATCC : 14990
- Souche Candida albicans, n°ATCC : 18804

### 3.2 Préparation des solutions tampons

- Tampon n°4 : bicarbonate d'ammonium 50mM pH8/stockage 1 mois à +4°C Pour 50ml tampon : 197,6mg de bicarbonate qsp 50ml H₂O/pH=7,9 ; ajout environ 10µl de NH₄OH pour obtenir pH=8
- Tampon n°5' : TCEP 150mM/à préparer extemporanément/dilution en tampon n°4
- Tampon n°6 : IAA 150mM/à préparer extemporanément

Pour 1 ml tampon : 27,7mg d'IAA qsp 1ml tampon bicarbonate n°4

### 3.3 Matériel

- Centrifugeuse "APPLI 24"/Prolabo
- Tubes 1,5ml ; « Safe Lock » Eppendorf, réf : 0030120.086
- Centrifugeuse "paillasse"/Eppendorf/ref 5415C
- Spectrophotomètre UVIKON + cuves quartz 80µl
- Boites culture BMX : COS (réf. :43041) et SDA (réf. : 43555)
- Sonde à ultrasons « Hielscher » ; réf : PN-66-NNN
- Billes de lyse 0,1mm (petit diamètre) : « Zirconia/Silica beads »/Roth/N033.1
- Billes de lyse 1 mm (grand diamètre) : « Silibeads typ 1/1,3mm/réf: 4504/VWR
- Cavaliers /Dutscher/référence : 011870A

### 3.4 Protocole

### 3.4.1. Etapes liminaires 800, 802, 804 et 806

De manière pratique, l'on prépare d'abord les solutions tampons n°5' (dilution d'une solution de TCEP 500mM à 150mM en tampon n°4 bicarbonate) et n°6 (27,7mg/ml d'IAA dans du tampon n°4 bicarbonate).

Ensuite, l'on pèse, à l'aide d'une spatule « cuillère », 50mg de billes de 0,1mm de diamètre et 50mg de billes de 1mm de diamètre que l'on introduit dans un tube d'une contenance de 1,5 ml. Tel qu'indiqué supra, le mélange des billes de « petit diamètre » et de « grand diamètre » est désigné par la référence numérique 8061, toujours en figure 8.

L'on prélève une à trois colonies 110 à partir d'une boîte de Petri 100, que l'on met en suspension dans l'eau à l'étape 800. La concentration en bactéries de la suspension ainsi obtenue est estimée par les méthodes classiques de mesure de turbidité (mesure de l'absorption à 550nm). Un volume de suspension correspondant à 1.108 CFU est prélevé puis centrifugé à l'étape 802, à 4000rpm durant 5 minutes à température ambiante. A l'issue de cette étape de centrifugation 802, le culot est repris (EC5, CA16 et SE9) à l'étape 804 dans 100µL de la solution tampon n°4 (tube n°2), puis l'on ajoute, toujours à l'étape 804, 3,5 µl de TCEP à 150mM (tampon n°5') pour obtenir une concentration finale de 5 mM en TCEP au sein du tube n°2. L'on vortexe 2 secondes (puissance maximale) pour homogénéiser le contenu de ce tube n°2 et l'on pipette ce mélange afin de le transférer, à l'étape 806, dans le tube n°1 contenant les billes 8061. Le tube n°2 est alors éliminé.

### 3.4.2. Etapes 808, 810 et 812

Sont ensuite introduits dans le tube n°1, à l'étape 808 :
- 9µL de solution d'IAA à 150mM (tampon n°6) afin d'obtenir une molarité finale de 12,5mM ; et
- 10µl de trypsine à 1µg/µl.

Suite à cela, l'on vortex 2 secondes (puissance maximale) pour homogénéisation.

Un cavalier est placé sur le tube n°1 pour éviter qu'il ne s'ouvre lors de la lyse par sonication.

Ce tube n°1 est ensuite introduit dans un des orifices de la sonde Hielscher (les 6 orifices à l'extrémité de la sonde sont supposés être identiques selon le fournisseur), puis l'on décompte 30minutes (réglages Amplitude 100/Cycle 0,5) afin de permettre aux réactions de lyse, de réduction, d'alkylation et de digestion trypsique de se produire conjointement/simultanément. La température du mélange dans le tube n°1, lors de l'étape 810, atteint environ 50°C.

Le tube n°1 est ensuite enlevé de l'orifice de la sonde à l'aide d'un « levier » en tenant le tube par le cavalier, puis ce tube n°1 est refroidi, à l'étape 812, en le stockant 1 minute dans la glace afin de ramener la température du mélange du tube à température ambiante.

En résumé, l'étape principale 810 permet d'effectuer conjointement/simultanément :
**a. La lyse des bactéries** / **réduction des protéines** (le TCEP rompt les ponts disulfures) : obtention des protéines dénaturées/réduites
**b. L'alkylation** : étape de blocage des ponts disulfures des protéines par méthylation avec l'IAA
**c. La digestion trypsique des protéines** : obtention des peptides.

### 3.4.3. Arrêt de la digestion trypsique 814

L'arrêt de la digestion trypsique s'effectue par ajout d'acide formique (qsp pH inférieur à 4) dans le tube n°1, à l'étape 814. Comme indiqué précédemment, le fait d'abaisser le pH en-dessous de 4 inactive, de façon réversible, l'activité enzymatique de la trypsine.

Le pH de l'échantillon est d'environ 8 (pH vérifié au pH mètre/sonde spéciale micro-tubes) avant l'ajout de 0,5µl d'acide formique. Il est d'environ 3 après ajout de ce dernier à l'étape 814. Le mélange est ensuite vortexé 2 secondes (puissance maximale) pour homogénéisation.

### 3.4.4. Etapes 816, 818, 820 et 822

Les peptides obtenus à l'issue de l'étape principale 810 se trouvent dans le surnageant. Ce dernier est donc pipeté (une partie des billes de 0,1mm est souvent récupérée) et transféré, à l'étape 816, dans un autre tube (tube n°3), puis centrifugé, à l'étape 818, durant 30 minutes, à 15000g et à 4°C.

90µl de surnageant comprenant les peptides sont ensuite récupérés à l'étape 820 et introduits dans un autre tube (tube n°4) à l'étape 822. Ce dernier est stocké au congélateur à une température de -20°C.

### 3.5. Validation des résultats

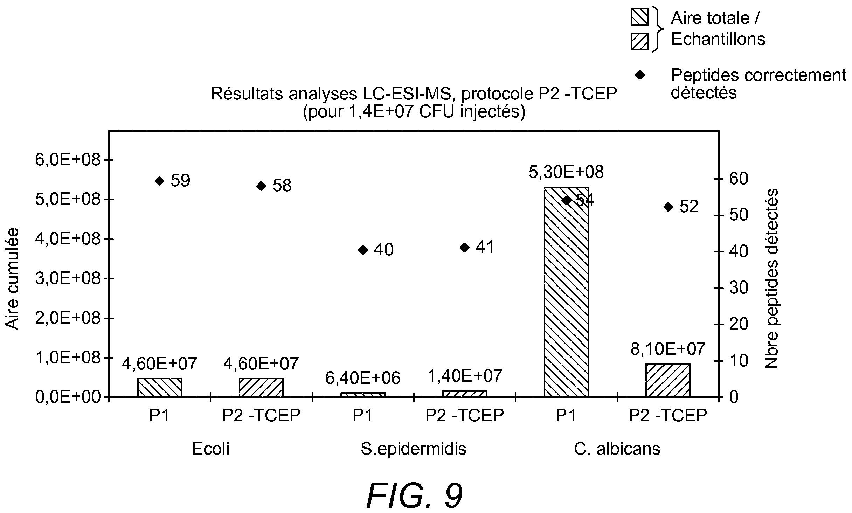

Les peptides contenus dans le tube n°4 sont ensuite analysés par LC-ESI-MS afin de déterminer la qualité/l'efficacité du protocole de lyse P2 (étape de validation des résultats). Les résultats, pour chacun des trois micro-organismes étudiés, sont présentés en figure 9. Ledit protocole P2 est désigné « P2-TCEP » sur cette figure 9, en référence au TCEP, utilisé en qualité d'agent dénaturant dans le présent exemple 3.

Comme expliqué précédemment, les valeurs représentées sur cette figure 9 correspondent aux nombres de peptides ciblés correctement détectés ainsi qu'à l'aire sous pic cumulée de ces peptides dans le spectre de masse obtenu par LC-ESI-MS en mode MRM.

Comme représenté sur cette figure 9, ce protocole P2 procure des résultats semblables à ceux obtenus par le protocole P1 susmentionné sur deux des trois micro-organismes testés, à savoir Escherichia Coli et Staphylococcus epidermidis. Les résultats obtenus pour Candida albicans sont satisfaisants au niveau du nombre de peptides.

Comme indiqué précédemment, les procédés (protocoles) d'obtention de peptides selon la présente invention s'inscrivent dans le cadre de la préparation d'échantillons de cellules procaryotes et/ou eucaryotes en vue de leur analyse ultérieure.

### Exemple 4 : Protocole P2 avec DTT (« P2-DTT »)

Le protocole P2 objet de l'exemple 3 supra a été reproduit en remplaçant le TCEP par un autre agent dénaturant, à savoir le DTT. A des fins de clarté, ce procédé P2 avec DTT est dénommé « P2-DTT ».

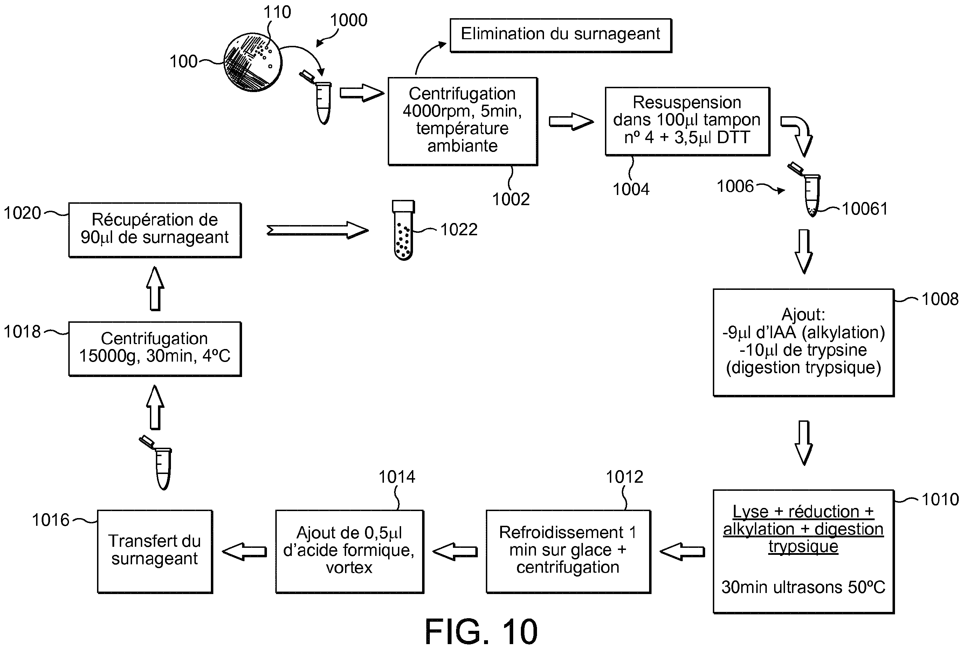

Le protocole P2-DTT est décrit ci-après, en référence à la figure 10.

Ce protocole P2-DTT donne des résultats semblables (analyses LC-ESI-MS) au protocole P1 décrit dans l'exemple 2, sur deux des trois micro-organismes testés : Escherichia Coli (Gram-) et Staphylococcus epidermidis (Gram+) tant en nombre de peptides qu'en aire cumulée.

### Matériel et méthode

### 4.1 Produits utilisés

- Acide formique /Fluka/référence : 06450
- Iodoacétamide, (IAA)/Sigma/I6125-5G/MM=184,96
- DL-1,4-Dithiothreitol 99%,
- (DTT)/Acros/165680010/lotA0269816/MM=154,24
- Ammonium bicarbonate/Sigma/A6141-500g/MM=79,06
- Hydroxyde d'ammonium,NH4OH/ammoniaque 28%/référence: 21190292/MM=17,03g
- Trypsine / Sigma/T0303 (stockage à -20°C)
- « Suspension médium » (eau stérile) bioMérieux (réf. : 70640)
- Souche Escherichia coli, n° ATCC : 11775T
- Souche Staphylococcus epidermidis, n°ATCC : 14990
- Souche Candida albicans, n°ATCC : 18804

### 4.2 Préparation des solutions tampons

- Tampon n°4 : bicarbonate d'ammonium 50mM pH8/stockage 1 mois à +4°C Pour 50ml tampon : 197,6mg de bicarbonate qsp 50ml H2O/pH=7,9 ; ajout environ 10µl de NH₄OH pour obtenir pH=8
- Tampon n°5 : DTT 150mM/à préparer extemporanément Pour 1ml de tampon : 23,1mg de DTT qsp 1ml tampon bicarbonate n°4
- Tampon n°6 : IAA 150mM/à préparer extemporanément Pour 1 ml tampon : 27,7mg d'IAA qsp 1ml tampon bicarbonate n°4

### 4.3 Matériel

- Centrifugeuse "APPLI 24"/Prolabo
- Tubes 1,5ml ; « Safe Lock » Eppendorf, réf : 0030120.086
- Centrifugeuse "paillasse"/Eppendorf/ref 5415C
- Spectrophotomètre UVIKON + cuves quartz 80µl
- Boites culture BMX : COS (réf. :43041) et SDA (réf. : 43555)
- Sonde à ultrasons « Hielscher » ; réf : PN-66-NNN
- Billes de lyse 0,1mm (petit diamètre) : « Zirconia/Silica beads »/Roth/N033.1
- Billes de lyse 1 mm (grand diamètre) : « Silibeads typ 1/1,3mm/réf : 4504/VWR
- Cavaliers /Dutscher/référence : 011870A

### 4.4 Protocole

### 4.4.1. Etapes liminaires 1000, 1002, 1004 et 1006

En premier lieu, l'on prépare les solutions tampons n°5 (DTT 150mM) et n°6 (IAA 150mM).

Ensuite, l'on pèse, à l'aide d'une spatule « cuillère », 50mg de billes de 0,1mm de diamètre et 50mg de billes de 1mm de diamètre que l'on introduit dans un tube d'une contenance de 1,5 ml. Tel qu'indiqué supra, le mélange des billes de « petit diamètre » et de « grand diamètre » est désigné par la référence numérique 10061, toujours en figure 10.

L'on prélève une à trois colonies 110 à partir d'une boîte de Petri 100, que l'on met en suspension dans l'eau à l'étape 1000. La concentration en bactéries de la suspension ainsi obtenue est estimée par les méthodes classiques de mesure de turbidité (mesure de l'absorption à 550nm). Un volume de suspension correspondant à 1.10⁸ CFU est prélevé puis centrifugé à l'étape 1002, à 4000rpm durant 5 minutes à température ambiante. A l'issue de cette étape de centrifugation 1002, le culot est repris (EC5, CA16 et SE9) à l'étape 1004 dans 100µL de la solution tampon n°4 (tube n°2), puis l'on ajoute, toujours à l'étape 1004, 3,5 µl de DTT à 150mM (tampon n°5) pour obtenir une concentration finale de 5 mM en DTT au sein du tube n°2. L'on vortexe 2 secondes (puissance maximale) pour homogénéiser le contenu de ce tube n°2 et l'on pipette ce mélange afin de le transférer, à l'étape 1006, dans le tube n°1 contenant les billes 10061. Le tube n°2 est alors éliminé.

### 4.4.2. Etapes 1008, 1010 et 1012

Sont ensuite introduits dans le tube n°1, à l'étape 1008 :
- 9µL de solution d'IAA à 150mM (tampon n°6) afin d'obtenir une molarité finale de 12,5mM; et
- 10µl de trypsine à 1µg/µl.

Suite à cela, l'on vortex 2 secondes (puissance maximale) pour homogénéisation.

Un cavalier est placé sur le tube n°1 pour éviter qu'il ne s'ouvre lors de la lyse par sonication.

Ce tube n°1 est ensuite introduit dans un des orifices de la sonde Hielscher (les 6 orifices à l'extrémité de la sonde sont supposés être identiques selon le fournisseur), puis l'on décompte 30minutes (réglages Amplitude 100/Cycle 0,5) afin de permettre aux réactions de lyse, de réduction, d'alkylation et de digestion trypsique de se produire conjointement/simultanément. La température du mélange dans le tube n°1, lors de l'étape 1010, atteint environ 50°C.

Le tube n°1 est ensuite enlevé de l'orifice de la sonde à l'aide d'un « levier » en tenant le tube par le cavalier, puis ce tube n°1 est refroidi, à l'étape 1012, en le stockant 1 minute dans la glace afin de ramener la température du mélange du tube à température ambiante.

En résumé, l'étape principale 1010 permet d'effectuer conjointement/simultanément :
**a. La lyse des bactéries** / **réduction des protéines** (le DTT rompt les ponts disulfures) : obtention des protéines dénaturées/réduites
**b. L'alkylation** : étape de blocage des ponts disulfures des protéines par méthylation avec l'IAA
**c. La digestion trypsique des protéines** : obtention des peptides.

### 4.4.3. Arrêt de la digestion trypsique 1014

L'arrêt de la digestion trypsique s'effectue par ajout d'acide formique (qsp pH inférieur à 4) dans le tube n°1, à l'étape 1014. Comme indiqué précédemment, le fait d'abaisser le pH en-dessous de 4 inactive, de façon réversible, l'activité enzymatique de la trypsine.

Le pH de l'échantillon est d'environ 8 (pH vérifié au pH mètre/sonde spéciale microtubes) avant l'ajout de 0,5µl d'acide formique. Il est d'environ 3 après ajout de ce dernier à l'étape 1014. Le mélange est ensuite vortexé 2 secondes (puissance maximale) pour homogénéisation.

### 4.4.4. Etapes 1016, 1018, 1020 et 1022

Les peptides obtenus à l'issue de l'étape principale 1010 se trouvent dans le surnageant. Ce dernier est donc pipeté (une partie des billes de 0,1mm est souvent récupérée) et transféré, à l'étape 1016, dans un autre tube (tube n°3), puis centrifugé, à l'étape 1018, durant 30 minutes, à 15000g et à 4°C.

90µl de surnageant comprenant les peptides sont ensuite récupérés à l'étape 1020 et introduits dans un autre tube (tube n°4) à l'étape 1022. Ce dernier est stocké au congélateur à une température de -20°C.

### 4.5. Validation des résultats

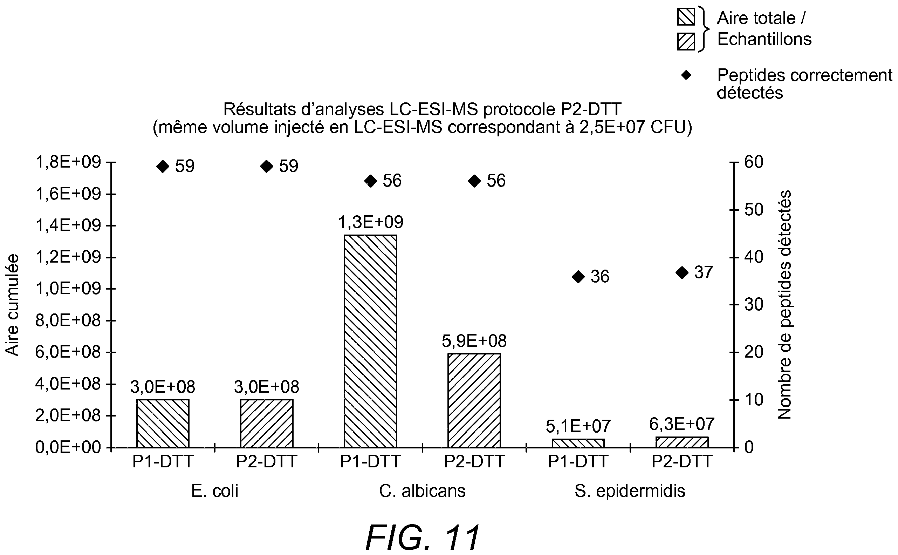

Les peptides contenus dans le tube n°4 sont ensuite analysés par LC-ESI-MS afin de déterminer la qualité/l'efficacité du protocole de lyse P2-DTT (étape de validation des résultats). Les résultats, pour chacun des trois micro-organismes étudiés, sont présentés en figure 11.

Comme expliqué précédemment, les valeurs représentées sur cette figure 11 correspondent aux nombres de peptides ciblés correctement détectés ainsi qu'à l'aire sous pic cumulée de ces peptides dans le spectre de masse obtenu par LC-ESI-MS en mode MRM.

Comme représenté sur cette figure 11, ce protocole P2 avec DTT procure des résultats semblables à ceux obtenus par le protocole P1 susmentionné sur deux des trois micro-organismes testés, à savoir Escherichia Coli et Staphylococcus epidermidis. Les résultats obtenus pour Candida albicans sont satisfaisants au niveau du nombre de peptides.

En outre, il s'avère que l'utilisation de DTT au lieu du TCEP comme agent dénaturant n'a pas d'impact visible tant au niveau du nombre de peptides que de l'aire cumulée globale.

Comme indiqué précédemment, les procédés (protocoles) d'obtention de peptides selon la présente invention s'inscrivent dans le cadre de la préparation d'échantillons de cellules procaryotes et/ou eucaryotes en vue de leur analyse ultérieure.

L'analyse de ces échantillons peut notamment permettre la recherche de biomarqueurs et peuvent être appliqués, entre autres, à l'analyse d'échantillons biologiques complexes tels que le plasma, l'urine, le liquide céphalorachidien, etc.

Dans le domaine de la microbiologie, les peptides obtenus par le procédé selon l'invention peuvent permettre la caractérisation d'au moins un microorganisme issu d'un échantillon, comprenant par exemple, l'identification dudit microorganisme et la détermination des propriétés de typage, résistance potentielle à au moins un antimicrobien et facteur de virulence concernant ledit micro-organisme.

Ce processus de caractérisation d'au moins un micro-organisme peut s'avérer particulièrement utile dans le domaine médical, pharmaceutique ou agroalimentaire.

La détermination des propriétés de typage, résistance à au moins un antimicrobien et facteur de virulence est mise en oeuvre par spectrométrie de masse en utilisant des protéines, peptides et/ou métaboliques en tant que marqueurs desdites propriétés de typage, résistance à au moins un antimicrobien et facteur de virulence. De préférence la spectrométrie de masse est de type MS/MS, avantageusement cette spectrométrie de masse est la MRM.

De préférence, la détermination des produits de typage, résistance à au moins un antimicrobien et facteur de virulence s'effectue dans le même appareil de spectrométrie de masse, simultanément.

De manière optimale, l'on peut envisager une spectrométrie de masse de type MS/MS, et avantageusement de type MRM, en tant qu'étape de confirmation de l'identification du microorganisme.

En effet, la caractérisation des micro-organismes est fondamentale tant dans le domaine clinique que dans le domaine industriel. Ainsi, par exemple, l'identification de résistances aux antimicrobiens tels que les antibiotiques, et la détection de facteurs de virulence sont des éléments essentiels pour assurer une prise en charge optimale des patients. De même, le typage est crucial pour les études épidémiologiques et pour assurer la lutte contre les maladies nosocomiales.

Par typage d'un micro-organisme, l'on entend la différentiation de plusieurs souches au sein d'une même espèce. Le typage a une valeur épidémiologique, le clinicien sait si la souche isolée chez le patient provient de la même source que d'autres souches apparemment identiques et isolées chez d'autres patients ou dans l'environnement. Cela permet ainsi de mettre en évidence un foyer infectieux au sein d'un hôpital ou lors d'une intoxication alimentaire. A titre d'exemples non limitatifs de marqueurs de propriétés de typage chez les bactéries, on peut citer des peptides présentant des mutations caractéristiques tels que les produits de transcription des gènes *adk, fum*C*, gyr*B*, icd, mdh, pur*A et *rec*A d' *Escherichia coli,* et ceux des gènes *arc, aro*E, *glp*F, *gmk, pta, tpi etyqi*L de *Staphylococcus aureus.* A titre d'exemples non limitatifs de marqueurs de propriétés de typage chez les protozoaires, on peut citer les produits du gène chitinase *d'Entamoeba histolytica* et *E. dispar.* A titre d'exemples non limitatifs de marqueurs de propriétés de typage chez les virus, on peut citer les produits du gène polymérase du virus de l'immunodéficience humaine. Enfin, à titre d'exemples non limitatifs de marqueurs de propriétés de typage chez les levures, on peut citer les produits de transcription des fragments de gènes *aat*1a, *acc*1, *adp*1, *mpi*b*, sya*1, *vps*13, et *zwf*1b de *Candida albicans.*

Par détermination de la résistance à au moins un antimicrobien, l'on entend la détermination de la susceptibilité d'un microorganisme à être détruit par un antimicrobien. Ainsi, si le microorganisme est une bactérie, l'antimicrobien contre lequel il peut développer une résistance est un antibiotique, si c'est un protozoaire, l'antimicrobien est un anti-parasitaire, si c'est un virus, l'antimicrobien est un antiviral, et, si c'est une levure, l'antimicrobien est un anti-fongique Les protéines impliquées dans les mécanismes de résistance vont différer selon la famille et l'espèce. A titre d'exemples non limitatifs de marqueurs de résistance à au moins un antibiotique utiles chez les bactéries, on peut citer les produits de transcription du gène *mec*A de *Staphylococcus aureus,* conférant une résistance à la Méticilline, et permettant d'indiquer si les souches sont méthicillino-résistantes (souches MRSA) ou bien méthillicino-sensibles (souches MSSA). On peut également citer la protéine TEM-2 qui permet d'indiquer si les souches d' *Escherichia coli* sont résistantes aux pénicillines mais sensibles à d'autres classes d'antibiotiques type céphalosporines ou carbapénèmes. Un autre marqueur est l'enzyme appelée KPC (pour *Klebsiella Pneumoniae* Carbapenemase) qui confère une résistance aux carbapénèmes. Un autre exemple de marqueur de résistance pour *Staphylococcus aureus* est le profil métabolique représentatif de la résistance à la vancomycine tel que décrit par Alexander E. et al dans le poster « Metabolomics-based approach to antibiotic resistance in Staphylococcus aureus » présenté au congrès de l'ASMS, 2009. A titre d'exemple non limitatif de marqueurs de résistance à au moins un anti-parasitaire utile chez les protozoaires, on peut citer la superoxide dismutase contenant du fer (Fe-SOD) et la péroxyrédoxine dont l'expression accrue confère une résistance au métronidazole. A titre d'exemple non limitatif de marqueur de résistance à au moins un antiviral utile chez les virus, on peut citer les mutations de l'enzyme transcriptase inverse du virus de l'immunodéficience humaine, conférant une sensibilité diminuée aux inhibiteurs nucléosidiques de la transcriptase inverse. Enfin, à titre d'exemple non limitatif de marqueurs de résistance à au moins un antifongique utile chez les levures, on peut citer la mutation de l'enzyme 1-3-b-D-glucane synthase de *Candida albicans,* conférant une sensibilité diminuée aux échinocandines. Pour autre exemple, on peut évoquer la résistance aux antifongiques azolés chez *Candida albicans,* en particulier la résistance au fluconazole. La cible du fluconazole est une enzyme, la lanostérol déméthylase, impliquée dans la synthèse de l'ergostérol, constituant principal de la paroi fongique. La résistance au fluconazole peut être associée à l'apparition de mutations ponctuelles dans le gène erg11 codant pour la lanostérol déméthylase.

Par détermination de la virulence d'un micro-organisme, l'on entend l'évaluation du caractère pathogène, nocif et violent du microorganisme. A titre d'exemples non limitatifs de marqueur de virulence chez les bactéries, on peut citer la PVL (Panton-Valentine Leucocidine), toxine cytolytique à deux composantes synergiques (LukFet LukS), présente chez *Staphylococcus aureus,* qui est l'une des toxines les plus virulentes causant des atteintes cutanées, de la cellulite extensive, de l'ostéomyélite et des pneumonies nécrosantes, et est impliquée dans des surinfections virales. D'autres exemples comprennent l'Autolysine et la Pneumolysine présents chez *Streptococcus pneumoniae,* espèce responsable d'infections des voies respiratoires, de méningites et de bactériémies, ainsi que les toxines A et B de *Clostridium difficile,* bactérie commensale de l'intestin, lesquelles provoquent soit l'altération de la perméabilité de l'épithélium intestinal (toxine A), soit s'attaquent directement aux cellules de l'épithélium (toxine B), soit diminuent dans le temps le transit intestinal et l'absorption intestinale, provoquant une diarrhée (action combinée des toxines A et B). On peut aussi citer comme exemple les Shiga toxines Stx1 et Stx2 présentes chez *Escherichia coli.* Ces deux cytotoxines sont considérés comme des facteurs de virulence importants des *Escherichia coli* entérohémorragiques. Elles sont responsables de complications comme la colite hémorragique ou le syndrome hémolytique urémique. A titre d'exemple non limitatif de marqueur de virulence chez les protozoaires, on peut citer des antioxydants (Fe-hydrogénase 2, peroxiredoxine, superoxyde dismutase) présents chez *Entamoeba histolytica,* espèce responsable de dysenterie, abcès hépatique. A titre d'exemple non limitatif de marqueur de virulence chez les virus, on peut citer le variant de la protéine Nef chez le virus de l'immunodéficience humaine de type 1, type plus pathogène chez l'être humain. Enfin, à titre d'exemple non limitatif de marqueur de virulence chez les levures, on peut citer la lipase 8 chez *Candida albicans,* espèce responsable de candidoses superficielles mais aussi de candidoses septicémiques et disséminées. Il convient de noter que les marqueurs spécifiques de la virulence sont également utilisables comme marqueur de typage.

A titre d'exemple de bactéries pouvant être caractérisées par un procédé d'analyse de ce type, l'on peut citer :
- *Escherichia coli* en utilisant TEM-2 comme marqueur de résistance et de typage, ainsi que Shiga toxins, OmpA comme marqueur de virulence et de typage.
- *Enterococcus faecalis* et *faecium* en utilisant VanA et VanB pour la résistance et le typage, ainsi que ESP (Enterococcal Surface Protein) pour la virulence et le typage, ou bien
- *Staphylococcus aureus* en utilisant la protéine appelée Immunoglobulin G-binding protéine A (également appelée protéine A) pour le typage, la protéine PBP2a pour la résistance, voire le typage, ainsi que la protéine PVL pour la virulence, voire également le typage.

A titre d'autres micro-organismes pouvant être caractérisés par le susdit procédé d'analyse, l'on peut citer :
- *Candida albicans* en utilisant l'enzyme 1-3-b-D-glucane synthase ou bien l'enzyme lanostérol déméthylase comme marqueur de résistance et de typage, ainsi que la lipase 8 comme marqueur de virulence et de typage.

Les cellules procaryotes et/ou eucaryotes selon la présente invention peuvent être obtenues à partir de tout échantillon susceptible de contenir un microorganisme cible. L'échantillon peut être d'origine biologique, soit animale, végétale ou humaine. Il peut alors correspondre à un prélèvement de fluide biologique (sang total, sérum, plasma, urine, liquide céphalo-rachidien, sécrétion organique, par exemple), un prélèvement tissulaire ou des cellules isolées. Ce prélèvement peut être utilisé tel quel dans la mesure où les marqueurs de caractérisation des microorganismes sont disponibles dans l'échantillon testé, ou bien il peut subir préalablement à l'analyse, une préparation de type enrichissement, extraction, concentration, purification, culture, selon des méthodes connues de l'homme du métier.

L'échantillon peut être d'origine industrielle, soit, selon une liste non exhaustive un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produit lacté (yaourts, fromages,), de viande, de poisson, d'oeuf, de fruit, de légume, d'eau, de boisson (lait, jus de fruits, soda, etc.). Ces échantillons d'origine alimentaire peuvent aussi provenir de sauces ou de plats élaborés. Un échantillon alimentaire peut enfin être issu d'une alimentation destinée aux animaux, telle que notamment des farines animales.

La spectrométrie de masse à mettre en oeuvre dans le procédé d'analyse selon l'invention est largement connue de l'homme du métier comme un outil puissant pour l'analyse et la détection de différents types de molécules. De façon générale, tout type de molécule pouvant être ionisée peut être détecté en fonction de sa masse moléculaire à l'aide d'un spectromètre de masse. Selon la nature de la molécule à détecter, d'origine protéique ou métabolique, certaines technologies de spectrométrie de masse peuvent être plus adaptées. Néanmoins, quelque soit la méthode de spectrométrie de masse utilisée pour la détection, cette dernière comprend une étape d'ionisation de la molécule cible en ions dits moléculaires, dans le cas présent une étape d'ionisation des marqueurs de caractérisation, et une étape de séparation des ions moléculaires obtenus en fonction de leur masse.

Tous les spectromètres de masse comportent donc :
i) une source d'ionisation destinée à ioniser les marqueurs présents dans l'échantillon à analyser, c'est-à-dire à conférer une charge positive ou négative à ces marqueurs;
ii) un analyseur de masse destiné à séparer les marqueurs ionisés, ou ions 15 moléculaires, en fonction de leur ratio masse sur charge (m /z) ;
iii) un détecteur destiné à mesurer le signal produit soit directement par les ions moléculaires, soit par des ions produits à partir des ions moléculaires, comme détaillés ci-après.

L'étape d'ionisation nécessaire pour la mise en oeuvre d'une spectrométrie de masse peut être effectuée par tout procédé connu de l'homme du métier. La source d'ionisation permet d'amener les molécules à doser sous un état gazeux et ionisé. Une source d'ionisation peut être utilisée soit en mode positif pour étudier les ions positifs, soit en mode négatif pour étudier les ions négatifs. Plusieurs types de sources existent et seront utilisés en fonction du résultat recherché et des molécules analysées. On peut citer, notamment :
- l'ionisation électronique (EI), l'ionisation chimique (CI) et la désorptionionisation chimique (DCI)
- le bombardement par atomes rapides (FAB), atomes métastables (MAB) ou ions (SIMS, LSIMS)
- le couplage plasma inductif (ICP)
- l'ionisation chimique à pression atmosphérique (APCI) et la photoionisation à pression atmosphérique (APPI)
- l'électronébulisation ou électrospray (ESI)
- la désorption-ionisation laser assistée par matrice MALDI), activée par une surface (SELDI) ou sur silicium (DIOS)
- l'ionisation-désorption par interaction avec espèces métastables (DART).

Notamment, l'ionisation peut être mise en oeuvre comme suit : l'échantillon contenant les molécules cibles est introduit dans une source d'ionisation, où les molécules sont ionisées à l'état gazeux et ainsi transformées en ions moléculaires qui correspondent aux molécules initiales. Une source d'ionisation de type électrospray (ESI pour ElectroSpray Ionisation) permet d'ioniser une molécule tout en la faisant passer d'un état liquide à un état gazeux. Les ions moléculaires obtenus correspondent alors aux molécules présentes à l'état liquide, avec en mode positif un, deux, voire trois protons supplémentaires ou plus et sont donc porteurs de une, deux, voire trois charges ou plus. Par exemple, lorsque la molécule cible est une protéine, une ionisation des peptides protéotypiques obtenus après fractionnement de la protéine cible, grâce à une source de type électrospray fonctionnant en mode positif, conduit à des ions polypeptidiques à l'état gazeux, avec un, deux, voire trois protons supplémentaires ou plus et qui sont donc porteurs de une, deux, voire trois charges ou plus, et permet un passage d'un état liquide à un état gazeux [18]. Ce type de source est particulièrement bien adapté, lorsque les molécules cibles ou peptides protéotypiques obtenus sont préalablement séparées par chromatographie liquide en phase inverse. Néanmoins, le rendement d'ionisation des molécules présentes dans l'échantillon peut varier en fonction de la concentration et de la nature des différentes espèces en présence. Ce phénomène se traduit par un effet matrice bien connu de l'homme de l'art.

Une source d'ionisation MALDI permettra d'ioniser des molécules, à partir d'un échantillon à l'état solide.

L'analyseur de masse dans lequel est mis en oeuvre l'étape de séparation des marqueurs ionisés en fonction de leur rapport masse/charge (m/z) est tout analyseur de masse connu de l'homme du métier. On peut citer les analyseurs basse résolution, du type quadripôle ou quadrupôle (Q), piège à ions 3D (IT) ou linéaire (LIT), également appelés trappe ionique, et les analyseurs haute résolution, permettant de mesurer la masse exacte des analytes et qui utilisent notamment le secteur magnétique couplé à un secteur électrique, le temps de vol (TOF).

La séparation des ions moléculaires en fonction de leur ratio m/z peut être mise en oeuvre une seule fois (spectrométrie de masse simple ou MS), ou bien plusieurs séparations MS successives peuvent être menées. Lorsque deux séparations MS successives sont réalisées, l'analyse est appelée MS/MS ou MS2. Lorsque trois séparations MS successives sont réalisées, l'analyse est appelée MS/MS/MS ou MS3 et plus généralement, lorsque n séparations MS successives sont réalisées, l'analyse est appelée MSn.

Parmi les techniques mettant en oeuvre plusieurs séparations successives, les modes SRM (Selected Reaction Monitoring) en cas de détection ou dosage d'une seule molécule cible, ou bien MRM (Multiple Reaction Monitoring) en cas de détection ou dosage de plusieurs molécules cibles, sont des utilisations particulières de séparation MS2. De même le mode MRM3 est une utilisation particulière de séparation en MS/MS/MS. On parle alors de spectrométrie de masse ciblée.

Dans le cas d'une détection en mode MS simple, c'est le rapport masse/charge des ions moléculaires obtenus qui est corrélé à la molécule cible à détecter.

Dans le cas d'une détection en mode MS/MS, essentiellement deux étapes sont ajoutées, par rapport à un dosage MS qui sont :
i) une fragmentation des ions moléculaires, alors appelés ions précurseurs, pour donner des ions dit ions fragments de 1ère génération, et
ii) une séparation des ions dit ions fragments de 1ère génération en fonction de leur masse (m/z)2, le rapport (m/z)1 correspondant au rapport (m/z) des ions précurseurs.

C'est alors le rapport masse/charge des ions fragments de 1ère génération ainsi obtenus qui est corrélé à la molécule cible à détecter. Par ion fragment de première génération, l'on entend un ion issu de l'ion précurseur, suite à une étape de fragmentation et dont le rapport masse sur charge m/z est différent de l'ion précurseur.

Les couples (m/z)1 et (m/z)2 sont baptisés transitions et sont représentatifs des ions caractéristiques à détecter.

Le choix des ions caractéristiques qui sont détectés pour être corrélés à la molécule cible est effectué par l'homme du métier selon les méthodes standards. Leur sélection conduira avantageusement aux dosages les plus sensibles, les plus spécifiques et les plus robustes possibles, en termes de reproductibilité et fiabilité. Dans les méthodes développées pour la sélection de peptides protéotypiques (m/z)₁, et de fragment de première génération (m/z)₂, le choix est essentiellement basé sur l'intensité de la réponse. Pour plus de détails, on pourra se référer à V. Fusaro et al. [4]. Des logiciels commerciaux, tels que les logiciels MIDAS et MRM Pilote d'Applied Biosytems ou encore MRMaid [5] pourront être utilisés par l'homme de l'art pour lui permettre de prédire tous les couples de transitions possibles. Il pourra également être fait appel à une base de données nommée PeptideAtlas, construite par F. Desiere et al. [6] pour compiler l'ensemble des transitions MRM de peptides décrites par la communauté scientifique. Cette base PeptideAtlas est disponible en accès libre sur internet.

Une approche alternative pour sélectionner les peptides protéotypiques (obtenus par exemple par digestion trypsique ; cf. supra), (m/z)₁ et (m/z)₂, consiste à utiliser les spectres de fragmentation MS/MS obtenus à l'occasion d'autres travaux. Ces travaux peuvent être, par exemple, les phases de découverte et d'identification des biomarqueurs par analyse protéomique. Cette approche a été proposée par Thermo Scientific lors de réunion utilisateurs [5]. Elle permet de générer une liste de transitions candidates à partir des peptides identifiés expérimentalement par le logiciel SIEVE (Thermo Scientific). Certains critères ont été détaillés par J. Mead et al. [5] pour le choix des ions (m/z)₁ et (m/z)₂ et sont détaillés ci-après :
- Les peptides avec des sites de clivage interne, c'est-à-dire avec de la Lysine ou de l'Arginine interne, doivent être évités, sauf si la Lysine ou l'Arginine est suivie par de la Proline,
- Les peptides avec de l'Asparagine ou de la Glutamine doivent être évités car ils peuvent se désaminer,
- Les peptides avec de la Glutamine ou de l'Acide Glutamique en N-terminal doivent être évités car ils peuvent se cycliser spontanément,
- Les peptides avec de la Méthionine doivent être évités car ils peuvent être oxydés,
- Les peptides avec de la Cystéine doivent être évités car ils peuvent être modifiés de façon non reproductible lors d'une éventuelle étape de dénaturation, réduction et blocage des fonctions thiols,
- Les peptides avec de la Proline peuvent être considérés comme favorables parce qu'ils produisent généralement des fragments intenses en MS/MS avec un seul pic très majoritaire. Cependant, un seul fragment très majoritaire ne permet pas de valider l'identité de la transition dans un mélange complexe. En effet, seule la présence simultanée de plusieurs fragments caractéristiques permet de vérifier que l'ion précurseur recherché est bien détecté,
- Les peptides ayant une Proline adjacente au C-terminal (position n-1) ou en seconde position par rapport au C-terminal (position n-2) sont à éviter car, dans ce cas, la taille du peptide fragment de première génération est généralement considérée comme trop petite pour être suffisamment spécifique,
- La sélection de fragments ayant une masse supérieure au précurseur est à privilégier pour favoriser la spécificité. Pour cela, il faut sélectionner un ion précurseur dichargé et sélectionner l'ion fragment de première génération le plus intense ayant une masse supérieure au précurseur, c'est à dire un ion fragment de première génération monochargé.

La fragmentation des ions précurseurs sélectionnés est mise en oeuvre dans une cellule de fragmentation telle que les modèles de type triple quadripôle [19], ou de type trappe ionique [20], ou encore de type temps de vol (TOF) [21], lesquels permettent également la séparation des ions. La ou les fragmentations seront classiquement réalisées par collision avec un gaz inerte tel que l'argon ou l'azote, au sein d'un champ électrique, par photo-excitation ou photodissociation à l'aide d'une source lumineuse intense, collision avec des électrons ou espèces radicalaires, par application d'une différence de potentiel, par exemple dans un tube de temps de vol, ou par tout autre mode d'activation. Les caractéristiques du champ électrique conditionnent l'intensité et la nature de la fragmentation. Ainsi, le champ électrique appliqué en présence d'un gaz inerte, par exemple dans un quadripôle, conditionne l'énergie de collision apportée aux ions. Cette énergie de collision sera optimisée, par l'homme du métier, pour accroître la sensibilité de la transition à doser. A titre d'exemple, il est possible de faire varier l'énergie de collision entre 5 et 180 e-V en q2 dans un spectromètre de masse AB SCIEX QTRAP® 5500 de la société Applied Biosystems (Foster City, Etats Unis d'Amérique). De même, la durée de l'étape de collision et l'énergie d'excitation au sein, par exemple, d'une trappe ionique seront optimisées, par l'homme du métier, pour conduire au dosage le plus sensible. A titre d'exemple, il est possible de faire varier cette durée, baptisée temps d'excitation, entre 0,010 et 50 ms et l'énergie d'excitation entre 0 et 1 (unité arbitraire) en Q3 dans un spectromètre de masse AB SCIEX QTRAP® 5500 de la société Applied Biosystems.

Enfin, la détection des ions caractéristiques sélectionnés se fait de façon classique, notamment grâce à un détecteur et à un système de traitement. Le détecteur collecte les ions et produit un signal électrique dont l'intensité dépend de la quantité d'ions collectée. Le signal obtenu est ensuite amplifié pour qu'il puisse être traité informatiquement. Un ensemble informatique de traitement des données permet de transformer les informations reçues par le détecteur en spectre de masse.

Le principe du mode SRM, ou encore du mode MRM, est de sélectionner spécifiquement un ion précurseur, de le fragmenter, puis de sélectionner spécifiquement l'un de ses ions fragments. Pour de telles applications, des dispositifs du type triple quadripôle ou des hybrides triple quadripôle à trappe ionique sont généralement utilisés.

Dans le cas d'un dispositif triple quadripôle (Q1q2Q3) utilisé en mode MS², en vue du dosage ou de la détection d'une protéine cible, le premier quadripôle (Q1) permet de filtrer les ions moléculaires, correspondant aux peptides protéotypiques caractéristiques de la protéine à doser et obtenus lors d'une étape antérieure de digestion, en fonction de leur ratio masse sur charge (m/z). Seuls les peptides ayant le ratio masse/charge du peptide protéotypique recherché, ratio appelé (m/z)₁, sont transmis dans le deuxième quadripôle (q2) et jouent le rôle d'ions précurseurs pour la fragmentation ultérieure. L'analyseur q2 permet de fragmenter les peptides de ratio masse/charge (m/z)₁ en ions fragments de première génération. La fragmentation est généralement obtenue par collision des peptides précurseurs avec un gaz inerte, comme de l'azote ou de l'argon dans q2. Les ions fragments de première génération sont transmis dans un troisième quadripôle (Q3) qui filtre les ions fragments de première génération en fonction d'un ratio masse sur charge spécifique, ratio appelé (m/z)₂.

Seuls les ions fragments de première génération ayant le ratio masse/charge d'un fragment caractéristique du peptide protéotypique recherché (m/z)₂ sont transmis dans le détecteur pour être détectés, voire quantifiés.

Ce mode de fonctionnement présente une double sélectivité, en relation avec la sélection de l'ion précurseur d'une part et de la sélection de l'ion fragment de première génération d'autre part. La spectrométrie de masse en mode SRM ou MRM est donc avantageuse pour la quantification

Lorsque la spectrométrie de masse mise en oeuvre dans le procédé de l'invention est une spectrométrie de masse en tandem (MS2, MS3, MS4 ou MS5), plusieurs analyseurs de masse peuvent être couplés entre eux. Par exemple, un premier analyseur sépare les ions, une cellule de collision permet de fragmenter les ions, et un second analyseur sépare les ions fragments. Certains analyseurs, comme les pièges à ions ou le FT-ICR, constituent plusieurs analyseurs en un et permettent de fragmenter les ions et d'analyser les fragments directement.

Selon des modes de réalisation préférés de l'invention, le procédé de l'invention comprend une ou plusieurs des caractéristiques suivantes :
- la spectrométrie de masse, mise en oeuvre pour les propriétés de typage, de résistance potentielle à au moins un antimicrobien et facteur de virulence, est une spectrométrie de type MS/MS, ce qui a pour avantage de générer un fragment spécifique de la molécule à détecter ou à quantifier, et ainsi d'apporter une grande spécificité à la méthode de dosage ;
- la spectrométrie MS/MS est de la MRM, ce qui a pour avantage d'utiliser un temps de cycle d'analyse dans le spectromètre de masse de quelques dizaines de millisecondes, ce qui permet de détecter ou de quantifier avec une grande sensibilité, et de façon multiplexée, un grand nombre de molécules différentes ;
- la détermination des propriétés de typage, résistance à un antimicrobien et facteur de virulence est mise en oeuvre dans le même appareil de spectrométrie de masse, de préférence simultanément, ce qui a pour avantage de réduire le temps d'analyse et le coût de l'instrument, cela facilite également le traitement et le rendu des résultats.

Outre la détermination des propriétés de typage, résistance à un antimicrobien et facteur de virulence, il convient d'identifier le ou les microorganismes présents dans l'échantillon à tester.

Les procédés d'identification de microorganismes sont largement connus de l'homme du métier, comme décrit par exemple par Murray P. R. el al. dans Manuel of Clinical Microbiology, 2007, 9ème édition, et en particulier dans le Vol. I, Section III, chapitres 15 et 16 pour les bactéries et levures, Vol. II, Section VI, chapitre 82 pour les virus, et Vol. II, Section X, chapitre 135 pour les protozoaires. A titre d'exemple de procédés classiques d'identification, on peut citer la détermination du profil biologique, en utilisant par exemple les cartes d'identification du Vitek 2 (bioMérieux), ou bien encore en utilisant des techniques de biologie moléculaire avec des critères d'identification basés sur l'étude de la présence de certains gènes, sur l'étude de leur séquence.

L'identification peut être mise en oeuvre directement à partir de l'échantillon dans lequel on effectue l'identification, ou bien les microorganismes contenus dans l'échantillon peuvent être cultivés par des méthodes bien connues de l'homme du métier avec des milieux de culture et des conditions de culture optimales adaptées en fonction des espèces de microorganismes à rechercher, comme décrit par Murray P.R. et al. dans Manuel of Clinical Microbiology, 2007, 9ème édition, Vol. I, Section III, chapitre 14, et en particulier dans le Vol. I, Section IV, chapitre 21 pour les bactéries, Vol. II, Section VI, chapitre 81 pour les virus, Vol. II, Section VIII, chapitre 117 pour les levures, et Vol. II, Section X, chapitre 134 pour les protozoaires.

Ainsi, de façon générale, dans le cas d'une identification par une méthode biochimique d'une bactérie dans un prélèvement, il faut d'abord l'obtenir en culture pure, par exemple après ensemencement sur gélose. La biologie moléculaire (PCR) peut dans certains cas s'appliquer directement à l'échantillon à analyser.

Au lieu de cultiver les microorganismes, ces derniers peuvent être concentrés par capture directement dans l'échantillon au moyen de surfaces actives. Un tel procédé a été décrit par W.-J. Chen et al. [1] qui ont capturé différentes espèces bactériennes à l'aide de billes magnétiques avec une surface activée au Fe₃O₄/TiO₂. Une capture par d'autres moyens est également possible, telle qu'une capture par des lectines [22], ou par des anticorps [23], ou encore par de la Vancomycine [24]. La capture permet de concentrer les microorganismes et ainsi de réduire ou même de supprimer l'étape de culture. Il s'en suit un gain de temps considérable.

L'identification peut également être mise en oeuvre par spectrométrie de masse, selon les techniques décrites précédemment, de préférence par MS, par MS/MS, ou bien par MS suivie d'une spectrométrie de type MS/MS, ce qui constitue un mode de réalisation de l'invention. Dans ce cas également, l'échantillon peut être soumis préalablement à une étape de culture telle qu'un ensemencement sur gélose.

L'utilisation d'un procédé d'identification par MS est avantageux car il peut être réalisé en quelques minutes et qu'il nécessite un spectromètre de masse avec un seul analyseur, c'est à dire un instrument moins complexe qu'un spectromètre de masse en tandem utilisé en MS/MS.

L'utilisation d'un procédé d'identification par MS suivi d'une spectrométrie de type MS/MS est également avantageuse. Elle permet de s'assurer de l'identité des ions observés en MS, ce qui augmente la spécificité de l'analyse.

L'utilisation d'un procédé d'identification par MS/MS de type MRM présente l'avantage d'être plus sensible et plus simple que les approches MS puis MS/MS traditionnelle. Ce procédé ne nécessite ni logiciel performant pour traiter l'information entre l'acquisition du spectre MS et du spectre MS/MS, ni changement du réglage des paramètres machines pour enchaîner des spectres MS puis MS/MS.

Le procédé d'identification par MS peut être mis en oeuvre avec une source électrospray sur échantillon brut, comme décrit par S. Vaidyanathan et al. [7] ou encore par R. Everley et al. [8] après séparation chromatographique. Différentes gammes de m/z permettent alors d'identifier les microorganismes. S. Vaidyanathan et al. ont utilisé une fenêtre entre 200 et 2000 Th et R. Everley et al. une fenêtre entre 620 et 2450 Th. Les spectres de masse peuvent également être déconvolués pour accéder à la masse des protéines indépendamment de leur état de charge. R. Everley et al. ont ainsi exploité les masses entre environ 5 000 et 50 000 Da. De façon alternative, le procédé d'identification par MS peut être également mis en oeuvre à l'aide d'un MALDI-TOF, comme décrit par Claydon et al [9] et T. Krishnamurthy et P. Ross [10]. L'analyse associe l'acquisition d'un spectre de masse et l'interprétation d'un logiciel expert. Elle est extrêmement simple et peut être effectuée en quelques minutes. Ce procédé d'identification se répand actuellement dans les laboratoires d'analyse médicale [25].

L'identification de bactéries par MS puis MS/MS via leurs protéines présentes dans l'échantillon, a été largement appliquée par de nombreuses équipes. A titre d'exemple, il est possible de citer les travaux récents de Manes N. et al. [11] qui ont étudié le peptidome de *Salmonelle enterica,* ou les travaux de R. Nandakumar et al. [12] ou de L. Hernychova et al. [13] qui ont étudié le protéome de bactéries après digestion des protéines avec de la trypsine. L'approche classique consiste à i) acquérir un spectre MS, ii) sélectionner successivement chaque ion précurseur observé sur le spectre MS avec un signal intense, iii) fragmenter successivement chaque ion précurseur et acquérir son spectre MS/MS, iv) interroger des bases de données protéiques telles que SWISS-PROT ou NCBI, aux travers de logiciels tels que Mascot (Matrix Science, Londres, Royaume-Uni) ou SEQUEST (Thermo Scientific, Waltham, Etats-Unis d'Amérique), pour identifier le peptide ayant une forte probabilité de correspondre au spectre MS/MS observé. Cette méthode peut conduire à l'identification d'un microorganisme si une protéine ou un peptide caractéristique de l'espèce est identifié.

Selon encore un autre mode de réalisation, l'identification dudit au moins un microorganisme est mis en oeuvre par un procédé classique d'identification et le procédé de l'invention comprend une étape supplémentaire de confirmation de l'identification dudit au moins un microorganisme, laquelle étape de confirmation est mise en oeuvre par spectrométrie de masse, selon les techniques décrites précédemment pour l'identification de microorganismes.

Selon un mode de réalisation particulier, la spectrométrie de masse de l'étape de confirmation est un spectrométrie de masse de type MS/MS, de préférence une MRM.

Un des avantages de l'utilisation de la spectrométrie de masse réside en ce qu'elle est particulièrement utile pour quantifier des molécules, dans le cas présent les marqueurs des propriétés de typage, résistance à au moins un antimicrobien. Pour ce faire, on utilise l'intensité de courant détectée, laquelle est proportionnelle à la quantité de molécule cible. L'intensité de courant ainsi mesurée pourra servir de mesure quantitative permettant de déterminer la quantité de molécule cible présente, laquelle est caractérisée par son expression en unités du Système International (SI) de type mol/m³ ou kg/m³, ou par les multiples ou sous-multiples de ces unités, ou par les dérivées usuelles des unités SI, y compris leurs multiples ou sous-multiples. A titre d'exemple non limitatif, les unités telles que ng/ml ou fmol/l sont des unités caractérisant une mesure quantitative.

Un calibrage est néanmoins nécessaire pour pouvoir corréler l'aire du pic mesurée, correspondant à l'intensité de courant induit par les ions détectés, à la quantité de molécule cible à doser. Pour cela, les calibrages classiquement utilisés en spectrométrie de masse pourront être mis en oeuvre, dans le cadre de l'invention. Les dosages MRM sont classiquement calibrés à l'aide de standards externes ou, de préférence, à l'aide de standards internes tels que décrits par T. Fortin et al. [2]. Dans le cas où la molécule cible est un peptide protéotypique, permettant de doser une protéine d'intérêt, la corrélation entre la mesure quantitative et la quantité de peptide protéotypique cible, et par la suite de protéine d'intérêt, est obtenue en étalonnant le signal mesuré par rapport à un signal étalon pour lequel la quantité à doser est connue. L'étalonnage peut être réalisé au moyen d'une courbe d'étalonnage, par exemple obtenue par injections successives de peptide protéotypique étalon à différentes, concentrations (étalonnage externe), ou de façon préférentielle, par étalonnage interne en utilisant un peptide lourd, comme standard interne, par exemple conformément aux méthodes AQUA, QconCAT ou PSAQ détaillées ci-après. Par « peptide lourd », l'on entend un peptide correspondant au peptide protéotypique, mais dans lequel un ou plusieurs atomes de carbone 12 (¹²C) est (sont) remplacé(s) par du carbone 13 (¹³C), et/ou un ou plusieurs atomes d'azote 14 (¹⁴N) est (sont) remplacé(s) par de l'azote 15 (¹⁵N).

L'utilisation de peptides lourds, comme standards internes (AQUA), a également été proposée dans la demande de brevet US 2004/0229283. Le principe est de synthétiser artificiellement des peptides protéotypiques avec des acides aminés comportant des isotopes plus lourds que les isotopes naturels usuels. De tels acides aminés sont obtenus, par exemple, en remplaçant certains des atomes de carbone 12 (¹²C) par du carbone 13 (¹³C), ou en replaçant certains des atomes d'azote 14 (¹⁴N) par de l'azote 15 (¹⁵N). Le peptide artificiel (AQUA) ainsi synthétisé a rigoureusement les mêmes propriétés physicochimiques que le peptide naturel (à l'exception d'une masse plus élevée). Il est généralement ajouté, à une concentration donnée, à l'échantillon, en amont du dosage par spectroscopie de masse, par exemple entre le traitement entrainant le clivage des protéines de l'échantillon d'intérêt et le fractionnement des peptides obtenus après l'étape de traitement. De ce fait, le peptide AQUA est co-purifié avec le peptide naturel à doser, lors du fractionnement des peptides. Les deux peptides sont donc injectés simultanément dans le spectromètre de masse, pour le dosage. Ils subissent alors les mêmes rendements d'ionisation dans la source. La comparaison des aires de pic des peptides naturels et AQUA, dont la concentration est connue, permet de calculer la concentration du peptide naturel et de remonter ainsi à la concentration de la protéine à doser. Une variante de la technique AQUA a été proposée par J.-M. Pratt et al. [14] sous le nom de QconCat. Cette variante est également décrite dans la demande de brevet WO 2006/128492. Elle consiste à concaténer différents peptides AQUA et à produire le polypeptide artificiel sous forme de protéine recombinante lourde. La protéine recombinante est synthétisée avec des acides aminés comportant des isotopes lourds. De cette façon, il est possible d'obtenir un standard pour calibrer le dosage simultané de plusieurs protéines à moindre coût. Le standard QconCAT est ajouté dès le début, en amont du traitement entrainant le clivage des protéines et avant les étapes de fractionnement des protéines, de dénaturation, de réduction puis de blocage des fonctions thiols des protéines, si celles-ci sont présentes. Le standard QconCAT subit donc le même cycle de traitement entrainant le clivage des protéines que la protéine naturelle, ce qui permet de tenir compte du rendement de l'étape de traitement entrainant le clivage des protéines. En effet, le traitement, notamment par digestion, de la protéine naturelle peut ne pas être complet. Dans ce cas l'utilisation d'un standard AQUA conduirait à sous-estimer la quantité de protéine naturelle. Pour un dosage absolu, il peut donc être important de tenir compte des rendements de traitement entrainant le clivage des protéines. Cependant, V. Brun et al. [15] ont montré que, parfois, les standards QconQAT ne reproduisaient pas exactement le rendement de traitement notamment par digestion de la protéine naturelle, sans doute du fait d'une conformation tridimensionnelle différente de la protéine QconCAT.

V. Brun et al. [15] ont alors proposé d'utiliser une méthode baptisée PSAQ et décrite dans la demande de brevet WO 2008/145763. Dans ce cas, le standard interne est une protéine recombinante, ayant la même séquence que la protéine naturelle mais synthétisée avec des acides aminés lourds. La synthèse est réalisée ex-vivo avec des acides aminés lourds. Ce standard a rigoureusement les mêmes propriétés physicochimiques que la protéine naturelle (à l'exception d'une masse plus élevée). Il est ajouté dès le début, avant l'étape de fractionnement des protéines, lorsque cette dernière est présente. Il est donc co-purifié avec la protéine native, lors de l'étape de fractionnement des protéines. Il présente le même rendement de traitement, notamment par digestion, que la protéine native. Le peptide lourd obtenu après clivage est également co-purifié avec le peptide naturel, si une étape de fractionnement des peptides est réalisée. Les deux peptides sont donc injectés simultanément dans le spectromètre de masse, pour être dosé quantitativement. Ils subissent alors les mêmes rendements d'ionisation dans la source. La comparaison des aires de pic des peptides naturels et des peptides de référence dans la méthode PSAQ permet de calculer la concentration de la protéine à doser en tenant compte de la totalité des étapes du procédé de dosage.

L'ensemble de ces techniques, à savoir AQUA, QconCAT ou PSAQ ou toute autre technique de calibrage, utilisée dans des dosages par spectrométrie de masse et en particulier dans les dosages MRM ou MS, pourront être mises en oeuvre pour effectuer le calibrage.

### Références bibliographiques

[1] W.-J. Chen et al., 2008, Anal. Chem., 80 : 9612-9621
[2] T. Fortin et al., 2009, Mol. Cell Proteomics, 8(5) : 1006-1015.
[3] H. Keshishian et al., 2007, Mol. Cell Proteomics, 2212-2229.
[4] V. Fusaro et al., 2009, Nature Biotech. 27, 190-198.
[5] J. Mead et al., 15 nov 2008, Mol. Cell Proteomics, E-pub.
[6] F. Desiere et al., 2006, Nucleic Acids Res., 34(database issue) : D655-8).
[7] S. Vaidyanathan et al., 2001, Anal. Chem., 73 :4134-4144.
[8] R. Everley et al., 2009, J. Microbiol. Methods, 77:152-158.
[9] M. Claydon et al, 1996, Nature Biotech. 14 :1584-1586.
[10] T. Krishnamurthy & P. Ross, 1996, Rapid Com. Mass Spec., 10 :1992-1996.
[11] Manes N. et al., 2007, Mol. & Cell. Proteomics, 6(4): 717-727.
[12] R. Nandakumar et al., 2009, Oral Microbiology Immunology, 24 :347-352).
[13] L. Hernychova et al., 2008, Anal. Chem., 80 :7097-7104.
[14] J.-M. Pratt et al., 2006, Nat. Protoc., 1:1029-1043.
[15] V. Brun et al., 2007, Mol. Cell Proteomics, 2139-2149.
[16] D. Lopez-Ferrer et al., 2008, Anal. Chem., 80 :8930-8936
[17] D. Lopez-Ferrer et al., 2005, J. Proteome res., 4(5) : 1569-1574
[18] Gaskell, Electrospray: principles and practise, 1997, J. Mass Spectrom., 32, 677-688).
[19] L. Anderson & C. Hunter, 2006, Mol. Cell Proteomics, 573-588).
[20] B. Han & R. Higgs, 2008, Brief Funct Genomic Proteomic.,7(5):340-54).
[21] K.-Y. Wang et al., 2008, Anal Chem, 80(16) 6159-6167).
[22] J. Bundy & C. Fenselau, 1999, Anal. Chem. 71 : 1460-1463.
[23] K-C Ho et al., 2004, Anal. Chem. 76 : 7162-7268.
[24] Y.S. Lin et al., 2005, Anal. Chem., 77 : 1753-1760.
[25] P. Seng et al., 2009, Clin. Infect. Dis., 49 :543-551.
[26] Santos H. M. et al. : «An improved clean sonoreactor-based method for protein identification by mass spectrometry-based techniques», TALANTA, ELSEVIER, AMSTERDAM, NL (2008). Vol. 77, no. 2, pages 870-875.

## Revendications

1. Procédé d'obtention de peptides à partir de cellules procaryotes et/ou eucaryotes, ledit procédé comprenant les étapes suivantes :
a) lyse des cellules procaryotes et/ou eucaryotes et récupération des protéines ainsi obtenues,
b) dénaturation desdites protéines en utilisant au moins un agent dénaturant,
c) alkylation des protéines dénaturées en utilisant au moins un agent alkylant,
d) protéolyse enzymatique des protéines obtenues à l'issue de l'étape c) en utilisant au moins une enzyme protéolytique,
e) récupération des peptides obtenus à l'issue de l'étape de protéolyse enzymatique d),
dans lequel la lyse des cellules procaryotes et/ou eucaryotes à l'étape a) est une lyse à faible concentration en agent(s) chaotropique(s),
et dans lequel au moins les étapes a) et b) sont effectuées conjointement ;
ladite lyse à faible concentration en agent(s) chaotropique(s) étant réalisée à une concentration en agent(s) chaotropique(s) inférieure ou égale à 1M, de préférence inférieure ou égale à 100 mM, et
ledit procédé étant mis en oeuvre sous une pression inférieure à 100 bars, de préférence inférieure à 50 bars, avantageusement inférieure à 10 bars.

2. Procédé selon la revendication 1, dans lequel la lyse à faible concentration en agent(s) chaotropique(s) est réalisée en l'absence d'agent(s) chaotropique(s) non salin(s) tel(s) que l'urée.

3. Procédé selon la revendication 1 ou 2, dans lequel la lyse à faible concentration en agent(s) chaotropique(s) est une lyse par sonication effectuée au moyen d'une sonde à ultrasons.

4. Procédé selon la revendication 3, dans lequel ladite sonde à ultrasons ne subit pas d'étape de préchauffage en préalable à la lyse par sonication.

5. Procédé selon l'une des revendications 1 à 4, dans lequel l'enzyme protéolytique est une protéase à sérine, préférablement sélectionnée dans le groupe consistant en la trypsine, la chymotrypsine et l'élastase avantageusement ladite enzyme protéolytique est la trypsine.

6. Procédé selon l'une des revendications 1 à 5, dans lequel l'agent dénaturant est un agent réducteur de thiols, de préférence sélectionné parmi le 2-mercaptoéthanol, le tris(2-carboxyethyl)phosphine, le dithioerythritol, la tributylphosphine et le dithiothreitol, avantageusement ledit agent réducteur de thiols est le tris(2-carboxyethyl)phosphine ou le dithiothreitol.

7. Procédé selon l'une des revendications 1 à 6, dans lequel l'agent alkylant est sélectionné dans le groupe constitué par la N-éthylmaléimide, l'iodoacétamide et la M-biotine, préférablement ledit agent alkylant est l'iodoacétamide.

8. Procédé selon l'une des revendications 1 à 7, dans lequel la durée minimale correspondant à l'étape de protéolyse d) est d'environ 15 minutes.

9. Procédé selon l'une des revendications 1 à 8, dans lequel au moins les étapes a) - c) sont effectuées conjointement.

10. Procédé selon l'une des revendications 1 à 9, dans lequel les étapes a) - d) sont effectuées conjointement.

11. Procédé selon la revendication 9 ou 10, dans lequel :
- l'agent dénaturant est un agent réducteur de thiols sélectionné parmi le tris(2-carboxyethyl)phosphine et le dithiothreitol, avantageusement ledit agent réducteur de thiols étant le tris(2-carboxyethyl)phosphine, et
- l'agent alkylant est l'iodoacétamide.

12. Procédé selon l'une des revendications 1 à 11, ledit procédé étant mis en oeuvre sous pression atmosphérique.

13. Procédé d'analyse de peptides de cellules procaryotes et/ou eucaryotes comprenant les étapes suivantes :
i) Obtention des peptides à partir desdites cellules procaryotes et/ou eucaryotes en utilisant le procédé selon l'une des revendications 1 à 12,
ii) Analyse des peptides ainsi obtenus, ladite analyse étant effectuée à l'aide d'un moyen d'analyse de type spectrométrie de masse.

14. Kit d'obtention de peptides à partir de cellules procaryotes et/ou eucaryotes, ledit kit étant spécifiquement adapté à la mise en oeuvre du procédé selon l'une des revendications 1 à 12, ledit kit comprenant :
- un nécessaire de lyse permettant d'effectuer ladite lyse à faible concentration en agent(s) chaotropique(s) des cellules procaryotes et/ou eucaryotes, de préférence un nécessaire de lyse par sonication, ledit nécessaire de lyse permettant en outre d'effectuer la lyse sous une pression inférieure à 100 bars, de préférence inférieure à 50 bars, avantageusement inférieure à 10 bars, et de manière particulièrement préférée sous pression atmosphérique,
- une première solution comprenant au moins un agent dénaturant, de préférence sélectionné parmi le 2-mercaptoéthanol, le tris(2-carboxyethyl)phosphine, le dithioerythritol, la tributylphosphine et le dithiothreitol, avantageusement ledit agent dénaturant est le tris(2-carboxyethyl)phosphine ou le dithiothreitol,
- une deuxième solution comprenant au moins un agent alkylant, de préférence sélectionné dans le groupe constitué par la N-éthylmaléimide, l'iodoacétamide et la M-biotine, préférablement ledit agent alkylant est l'iodoacétamide,
- une troisième solution comprenant au moins une enzyme protéolytique telle qu'une protéase à sérine, préférablement sélectionnée dans le groupe consistant en la trypsine, la chymotrypsine et l'élastase, avantageusement ladite enzyme protéolytique est la trypsine.

15. Utilisation du kit selon la revendication 14 pour la mise en oeuvre du procédé selon l'une des revendications 1 à 13.

## Patentansprüche

1. Ein Verfahren zum Erhalten von Peptiden aus prokaryotischen und/oder eukaryotischen Zellen, wobei das Verfahren die folgenden Schritte beinhaltet:
a) Lysieren der prokaryotischen und/oder eukaryotischen Zellen und Rückgewinnen der so gewonnenen Proteine,
b) Denaturieren dieser Proteine unter Verwendung von mindestens einem Denaturierungsmittel,
c) Alkylieren der denaturierten Proteine unter Verwendung von mindestens einem Alkylierungsmittel,
d) enzymatisches Proteolysieren der am Ende von Schritt c) erhaltenen Proteine unter Verwendung von mindestens einem proteolytischen Enzym,
e) Rückgewinnen der am Ende von dem enzymatischen Proteolyseschritt d) erhaltenen Peptide,
wobei die Lyse der prokaryotischen und/oder eukaryotischen Zellen in Schritt a) eine Lyse bei niedriger Konzentration an chaotropem/chaotropen Mittel(n) ist,
und wobei mindestens die Schritte a) und b) gleichzeitig durchgeführt werden;
die Lyse bei niedriger Konzentration an chaotropem/chaotropen Mittel(n) bei einer Konzentration an chaotropem/chaotropen Mittel(n) unterhalb von oder gleich 1 M, vorzugsweise unterhalb von oder gleich 100 mM verwirklicht wird, und
wobei das Verfahren unter einem Druck unterhalb von 100 bar, vorzugsweise unterhalb von 50 bar, besonders bevorzugt unterhalb von 10 bar ausgeführt wird.

2. Verfahren gemäß Anspruch 1, wobei das Lysieren bei niedriger Konzentration an chaotropem/chaotropen Mittel(n) bei Abwesenheit von nicht salzartigem/nicht salzartigen chaotropem/chaotropen Mittel(n) wie Harnstoff verwirklicht wird.

3. Verfahren gemäß Anspruch 1 oder 2, wobei das Lysieren bei niedriger Konzentration an chaotropem/chaotropen Mittel(n) ein Lysieren durch mittels einer Ultraschallsonde durchgeführter Ultraschallbehandlung ist.

4. Verfahren gemäß Anspruch 3, wobei die Ultraschallsonde dem Lysieren durch Ultraschallbehandlung keinem vorausgehenden Vorwärmungsschritt unterzogen wird.

5. Verfahren gemäß einem der Ansprüche 1 bis 4, wobei das proteolytische Enzym eine Serinprotease ist, bevorzugt ausgewählt aus der Gruppe, bestehend aus Trypsin, Chymotrypsin und Elastase, wobei das proteolytische Enzym besonders bevorzugt Trypsin ist.

6. Verfahren gemäß einem der Ansprüche 1 bis 5, wobei das Denaturierungsmittel ein Reduktionsmittel für Thiole ist, vorzugsweise ausgewählt aus 2-Mercaptoethanol, Tris(2-carboxyethyl)phosphin, Dithioerythritol, Tributylphosphin und Dithiothreitol, wobei das Reduktionsmittel für Thiole besonders bevorzugt Tris(2-carboxyethyl)phosphin oder Dithiothreitol ist.

7. Verfahren gemäß einem der Ansprüche 1 bis 6, wobei das Alkylierungsmittel ausgewählt ist aus der Gruppe, die aus N-Ethylmaleimid, lodacetamid und M-Biotin besteht, wobei das Alkylierungsmittel bevorzugt lodacetamid ist.

8. Verfahren gemäß einem der Ansprüche 1 bis 7, wobei die minimale, dem Proteolyseschritt d) entsprechende Zeitdauer ungefähr 15 Minuten beträgt.

9. Verfahren gemäß einem der Ansprüche 1 bis 8, wobei mindestens die Schritte a)-c) gleichzeitig durchgeführt werden.

10. Verfahren gemäß einem der Ansprüche 1 bis 9, wobei die Schritte a)-d) gleichzeitig durchgeführt werden.

11. Verfahren gemäß Anspruch 9 oder 10, wobei:
- das Denaturierungsmittel ein Reduktionsmittel für Thiole ist, ausgewählt aus Tris(2-carboxyethyl)phosphin und Dithiothreitol, wobei das Reduktionsmittel für Thiole besonders bevorzugt Tris(2-carboxyethyl)phosphin ist, und
- das Alkylierungsmittel lodacetamid ist.

12. Verfahren gemäß einem der Ansprüche 1 bis 11, wobei das Verfahren unter Atmosphärendruck ausgeführt wird.

13. Ein Verfahren zur Analyse von Peptiden aus prokaryotischen und/oder eukaryotischen Zellen, beinhaltend die folgenden Schritte:
i) Erhalten der Peptide aus den prokaryotischen und/oder eukaryotischen Zellen unter Verwendung des Verfahrens gemäß einem der Ansprüche 1 bis 12,
ii) Analysieren der so erhaltenen Peptide, wobei das Analysieren mithilfe eines Analysemittels der Massenspektrometrie-Art durchgeführt wird.

14. Ein Kit zum Erhalten von Peptiden aus prokaryotischen und/oder eukaryotischen Zellen, wobei das Kit spezifisch zur Ausführung des Verfahrens gemäß einem der Ansprüche 1 bis 12 angepasst ist, wobei das Kit Folgendes beinhaltet:
- einen Lysesatz, der es erlaubt, die Lyse von prokaryotischen und/oder eukaryotischen Zellen bei niedriger Konzentration an chaotropem/chaotropen Mittel(n) durchzuführen, vorzugsweise einen Satz für Lyse durch Ultraschallbehandlung, wobei der Lysesatz des Weiteren erlaubt, die Lyse unter einem Druck unterhalb von 100 bar, vorzugsweise unterhalb von 50 bar, besonders bevorzugt unterhalb von 10 bar, und ganz besonders bevorzugt bei Atmosphärendruck durchzuführen,
- eine erste Lösung, beinhaltend mindestens ein Denaturierungsmittel, vorzugsweise ausgewählt aus 2-Mercaptoethanol, Tris(2-carboxyethyl)phosphin, Dithioerythritol, Tributylphosphin und Dithiothreitol, wobei das Denaturierungsmittel besonders bevorzugt Tris(2-carboxyethyl)phosphin oder Dithiothreitol ist,
- eine zweite Lösung, beinhaltend mindestens ein Alkylierungsmittel, vorzugsweise ausgewählt aus der Gruppe, die aus N-Ethylmaleimid, lodacetamid und M-Biotin besteht, wobei das Alkylierungsmittel vorzugsweise lodacetamid ist,
- eine dritte Lösung, beinhaltend mindestens ein proteolytisches Enzym wie eine Serinprotease, bevorzugt ausgewählt aus der Gruppe, bestehend aus Trypsin, Chymotrypsin und Elastase, wobei das proteolytische Enzym besonders bevorzugt Trypsin ist.

15. Eine Verwendung des Kits gemäß Anspruch 14 zur Ausführung des Verfahrens gemäß einem der Ansprüche 1 bis 13.

## Claims

1. Method of obtaining peptides from procaryotic and/or eucaryotic cells, said method comprising the following steps:
a) lysis of the procaryotic and/or eucaryotic cells and recovery of the proteins thus obtained,
b) denaturation of said proteins using at least one denaturing agent,
c) alkylation of the denatured proteins using at least one alkylating agent,
d) enzymatic proteolysis of the proteins obtained at the end of step c) using at least one proteolytic enzyme,
e) recovery of the peptides obtained at the end of the enzymatic proteolysis step d), in which the lysis of the procaryotic and/or eucaryotic cells in step a) is a lysis at a low concentration of chaotropic agent(s);
and in which at least steps a) and b) are performed conjointly;
said lysis at a low concentration of chaotropic agent(s) being carried out at a concentration of chaotropic agent(s) less than or equal to 1 M, preferably less than or equal to 100 mM, and
said method being implemented under a pressure below 100 bar, preferably less than 50 bar, advantageously less than 10 bar.

2. Method according to claim 1, in which the lysis at a low concentration of chaotropic agent(s) is carried out in the absence of non-saline chaotropic agent(s) such as urea.

3. Method according to claim 1 or 2, in which the lysis at a low concentration of chaotropic agent(s) is a lysis by sonication performed by means of an ultrasound probe.

4. Method according to claim 3, in which said ultrasound probe is not subjected to a preheating step prior to lysis by sonication.

5. Method according to one of claims 1 to 4, in which the proteolytic enzyme is a serine protease preferably selected from the group consisting of trypsin, chymotrypsin and elastase advantageously said proteolytic enzyme is trypsin.

6. Method according to one of claims 1 to 5, in which the denaturing agent is a thiol-reducing agent, preferably selected from 2-mercaptoethanol, tris(2-carboxyethyl)phosphine, dithioerythritol, tributylphosphine and dithiothreitol, advantageously said thiol-reducing agent is tris(2-carboxyethyl)phosphine or dithiothreitol.

7. Method according to one of claims 1 to 6, in which the alkylating agent is selected from the group consisting of N-ethylmaleimide, iodoacetamide and M-biotin, preferably said alkylating agent is iodoacetamide.

8. Method according to one of claims 1 to 7, in which the minimum duration corresponding to the step of proteolysis d) is about 15 minutes.

9. Method according to one of claims 1 to 8, in which at least steps a) - c) are performed conjointly.

10. Method according to one of claims 1 to 9, in which steps a) - d) are performed conjointly.

11. Method according to claim 9 or 10, in which:
- the denaturing agent is a thiol-reducing agent selected from tris(2-carboxyethyl)phosphine and dithiothreitol, advantageously said thiol-reducing agent being tris(2-carboxyethyl)phosphine, and
- the alkylating agent is iodoacetamide.

12. Method according to one of claims 1 to 11, said method being implemented under atmospheric pressure.

13. Method of analysis of peptides of procaryotic and/or eucaryotic cells comprising the following steps:
i) Obtaining peptides from said procaryotic and/or eucaryotic cells using the method according to one of claims 1 to 12,
ii) Analysis of the peptides thus obtained, said analysis being performed using an analysis means of mass spectrometry type.

14. Kit for obtaining peptides from procaryotic and/or eucaryotic cells, said kit being specifically suited to implementing the method according to one of claims 1 to 12, said kit comprising:
- a lysis set allowing said lysis to be performed at low concentration of chaotropic agent(s) of procaryotic and/or eucaryotic cells, preferably a set for lysis by sonication, said lysis set further making it possible to perform the lysis under a pressure below 100 bar, preferably less than 50 bar, advantageously less than 10 bar, and most preferably under atmospheric pressure,
- a first solution comprising at least one denaturing agent, preferably selected from 2-mercaptoethanol, tris(2-carboxyethyl)phosphine, dithioerythritol, tributylphosphine and dithiothreitol, advantageously said denaturing agent is tris(2-carboxyethyl)phosphine or dithiothreitol,
- a second solution comprising at least one alkylating agent, preferably selected from the group consisting of N-ethylmaleimide, iodoacetamide and M-biotin, preferably said alkylating agent is iodoacetamide,
- a third solution comprising at least one proteolytic enzyme such as a serine protease, preferably selected from the group consisting of trypsin, chymotrypsin and elastase, advantageously said proteolytic enzyme is trypsin.

15. Use of the kit according to claim 14 for implementing the method according to one of claims 1 to 13.
